# EUROPEAN PATENT APPLICATION

(11) **EP 4 755 916 A1**
(43) Date of publication of application: **10.06.2026**
(21) Application number: 24850932.5
(22) Date of filing: 02.08.2024
(51) Int. Cl.: C07K 16/28, A61K 39/395, A61P 35/00, A61P 35/02

(54) **BTN3A-BINDING PROTEIN AND MEDICAL USES THEREOF**

(30) Priority: 04.08.2023 CN 202310976510
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: WANG, Huan, Shanghai 201210 (CN); GAO, Han, Shanghai 201210 (CN); ZHENG, Xin, Shanghai 201210 (CN); ZHANG, Chongqi, Shanghai 201210 (CN); LIN, Yuan, Shanghai 201210 (CN); SU, Lu, Shanghai 201210 (CN); LIAO, Cheng, Lianyungang, Jiangsu 222047 (CN)
(74) Representative: Regimbeau
(86) International application number: PCT/CN2024/109487
(87) International publication number: WO 2025/031287

(57) **Abstract**

The present invention relates to a BTN3A-binding protein and medical uses thereof. Specifically, the present invention relates to the BTN3A-binding protein, a method thereof for activating γδ T cells and promoting cytokine release in γδ T cells, a method for treating cancer, and medical uses.

## Description

The present invention claims priority to the Chinese Patent Application No.: 2023109765101 entitled "BTN3A-BINDING PROTEIN AND MEDICAL USES THEREOF" and filed on August 4, 2023, which is incorporated herein by reference.

### Technical Field

The present invention relates to the field of biomedicine, and specifically to a BTN3A-binding protein, a method for activating γδ T cells and a method for treating cancer using same, as well as related pharmaceutical uses thereof.

### Background Art

The advent of immunotherapy has transformed the landscape of traditional cancer treatment. Most current immunotherapy approaches focus on enhancing the anti-tumor immune responses mediated by αβ T cells; however, there remain other lymphocyte subsets with anti-tumor potential that are worthy of attention. γδ T cells are a unique subset of T cells with innate immune characteristics, accounting for about 0.5-5% of total T cells in humans. They can be activated by directly recognizing antigen signals transmitted by specific ligands independent of the major histocompatibility complex (MHC) and antigen-presenting cells (APCs). All T cells express TCRs, and are classified into αβ T cells and γδ T cells, depending on differences in their TCR genes, where αβ T cells express Vα and Vβ TCR chains, and γδ T cells express Vγ and Vδ TCR chains. γδ T cells express a total of seven Vγ TCR chains (Vγ2, 3, 4, 5, 8, 9, and 11) and four Vδ TCR chains (Vδ1, 2, 3, and 5). Based on differences in their Vδ chains, human γδ T cells are classified into three major subsets: Vδ1 T cells, Vδ2 T cells, and Vδ3 T cells. Vδ1 T cells are primarily localized in mucosal epithelial tissues, Vδ2 T cells are mainly found in peripheral blood, and Vδ3 T cells are predominantly distributed in the liver and intestines. The Vγ9Vδ2 T cell subset, accounting for about 1-5% of total T cells in peripheral blood, is the most extensively studied subset to date. Studies have shown that activated Vγ9Vδ2 T cells can kill cancer cells by secreting cytokines such as IFN-γ and TNF-α, or by releasing perforin and granzymes. Additionally, activated Vγ9Vδ2 T cells proliferate simultaneously, thereby further exerting an anti-tumor effect.

In recent years, butyrophilin subfamily 3 member A (BTN3A) has emerged as a key molecule that regulates the function of Vγ9Vδ2 T cells. The BTN3A family includes three isoforms: BTN3A1 (also known as CD277), BTN3A2, and BTN3A3, which are respectively expressed by three distinct genes on human chromosome 6, and are expressed in both humans and non-human primates (NHPs). The BTN3A family belongs to type I transmembrane proteins. The extracellular segment of each BTN3A isoform consists of one IgV domain far from the cell membrane and one IgC domain close to the cell membrane, with the sequence homology of the extracellular segments among the three isoforms as high as 95%. The cytoplasmic regions of BTN3A1 and BTN3A3 comprise one B30.2 domain, which is absent from BTN3A2. Recent studies have found that under bacterial or viral stimulation and stress conditions, intracellularly accumulated phosphorylated antigens (pAgs) are recognized and bound by the B30.2 domain in the intracellular segment of BTN3A proteins. Upon binding to pAgs, the extracellular segment of BTN3A proteins undergoes a conformational change, which further promotes the formation of a complex with BTN2A1 proteins on the cell membrane surface. It then binds to the Vγ9 chain of Vγ9Vδ2 T cells via the IgV domain of BTN2A1, thereby activating Vγ9Vδ2 T cells to exert their functions. Due to the low affinity of BTN3A3 for pAgs, only BTN3A1 can sense an increase in intracellular pAgs and activate Vγ9Vδ2 T cells.

Recent studies have found that by designing antibodies targeting the extracellular segment of BTN3A1, intracellular pAgs can be replaced, enabling more efficient activation of Vγ9Vδ2 T cells. Furthermore, anti-BTN3A1 antibodies can enhance the anti-tumor activity of αβ T cells.

The present invention provides an anti-BTN3A antibody of novel sequence structure having an excellent activity of activating γδ T cells and promoting cytokine release by γδ T cells, which in turn enhances the killing of tumor cells by γδ T cells, thereby exhibiting good drugability and potential to be developed as a clinical drug.

### Summary of the Invention

The present invention provides a BTN3A-binding protein, a nucleic acid encoding same, a vector, a host cell, a pharmaceutical composition, and a method for activating γδ T cells and promoting cytokine release by γδ T cells, and a method for treating or preventing a disease (e.g., cancer) using same, as well as related pharmaceutical uses thereof.

### BTN3A-binding protein

The present invention provides a BTN3A-binding protein, comprising a domain that specifically binds to BTN3A and comprises a heavy chain variable region (VH) and/or a light chain variable region (VL). In some embodiments, the BTN3A-binding protein comprises one or more domains that specifically bind to BTN3A.

The present invention provides a BTN3A-binding protein, comprising a heavy chain variable region (VH) and/or a light chain variable region (VL).

In some embodiments, the heavy chain variable region comprises HCDR1, HCDR2, and HCDR3 of the amino acid sequences as set forth in SEQ ID NOs: 17-19, respectively, and the light chain variable region comprises LCDR1, LCDR2, and LCDR3 of the amino acid sequences as set forth in SEQ ID NOs: 20-22, respectively, as follows.
HCDR1 (SEQ ID NO: 17): X₁YYX₂X₃, wherein X₁ is selected from D or N, X₂ is selected from I or M, and X₃ is selected from T or N;
HCDR2 (SEQ ID NO: 18): X₄IYGSX₅NTX₆YASWAKG, wherein X₄ is selected from V or I, X₅ is selected from S or D, and X₆ is selected from V or Y;
HCDR3 (SEQ ID NO: 19): X₇X₈X₉X₁₀SSAX₁₁X₁₂X₁₃X₁₄, wherein X₇ is selected from G or N, X₈ is selected from Y or L, X₉ is selected from L or D, X₁₀ is selected from A or Y, X₁₁ is selected from D or Y, X₁₂ is selected from I or F, X₁₃ is absent or H, and X₁₄ is absent or I;
LCDR1 (SEQ ID NO: 20): QX₁₅SQSVYNNNRLA, wherein X₁₅ is selected from S or A;
LCDR2 (SEQ ID NO: 21): X₁₆ASX₁₇LAS, wherein X₁₆ is selected from D or E, and X₁₇ is selected from T or K; and
LCDR3 (SEQ ID NO: 22): QX₁₈YYSGX₁₉IX₂₀X₂₁, wherein X₁₈ is selected from T or G, X₁₉ is selected from Y or F, X₂₀ is selected from W or Y, and X₂₁ is selected from A or P.

In some embodiments, the heavy chain variable region comprises HCDR1, HCDR2, and HCDR3 in a heavy chain variable region of the amino acid sequence as set forth in any one of SEQ ID NOs: 1, 3, 31-35, and 39-44, and the light chain variable region comprises LCDR1, LCDR2, and LCDR3 in a light chain variable region of the amino acid sequence as set forth in any one of SEQ ID NOs: 2, 4, 36-38, and 45-47.

In some embodiments, the heavy chain variable region comprises HCDR1, HCDR2, and HCDR3 in a heavy chain variable region of the amino acid sequence as set forth in any one of SEQ ID NOs: 1 and 31-35, and the light chain variable region comprises LCDR1, LCDR2, and LCDR3 in a light chain variable region of the amino acid sequence as set forth in any one of SEQ ID NOs: 2 and 36-38.

In some embodiments, the heavy chain variable region comprises HCDR1, HCDR2, and HCDR3 of the amino acid sequences as set forth in SEQ ID NOs: 5-7, respectively, and the light chain variable region comprises LCDR1, LCDR2, and LCDR3 of the amino acid sequences as set forth in SEQ ID NOs: 8-10, respectively.

In some embodiments, the heavy chain variable region comprises HCDR1, HCDR2, and HCDR3 in a heavy chain variable region of the amino acid sequence as set forth in any one of SEQ ID NOs: 3 and 39-44, and the light chain variable region comprises LCDR1, LCDR2, and LCDR3 in a light chain variable region of the amino acid sequence as set forth in any one of SEQ ID NOs: 4 and 45-47.

In some embodiments, the heavy chain variable region comprises HCDR1, HCDR2, and HCDR3 of the amino acid sequences as set forth in SEQ ID NOs: 11-13, respectively, and the light chain variable region comprises LCDR1, LCDR2, and LCDR3 of the amino acid sequences as set forth in SEQ ID NOs: 14-16, respectively.

The above-mentioned CDRs are defined according to the Kabat, IMGT, Chothia, AbM, or Contact numbering system, e.g., the Kabat numbering system.

In some embodiments, the BTN3A-binding protein comprises a heavy chain variable region of the amino acid sequence as set forth in any one of SEQ ID NOs: 1, 3, 31-35, and 39-44, or an amino acid sequence with at least 80% or at least 90% identity thereto, and/or a light chain variable region of the amino acid sequence as set forth in any one of SEQ ID NOs: 2, 4, 36-38, and 45-47, or an amino acid sequence with at least 80% or at least 90% identity thereto.

In some embodiments, the BTN3A-binding protein comprises a heavy chain variable region of the amino acid sequence as set forth in any one of SEQ ID NOs: 1 and 31-35 or an amino acid sequence with at least 80% or at least 90% identity thereto, and a light chain variable region of the amino acid sequence as set forth in any one of SEQ ID NOs: 2 and 36-38 or an amino acid sequence with at least 80% or at least 90% identity thereto.

In some specific embodiments, the BTN3A-binding protein comprises:
a heavy chain variable region of the amino acid sequence as set forth in SEQ ID NO: 1 or an amino acid sequence with at least 80% or at least 90% identity thereto, and a light chain variable region of the amino acid sequence as set forth in SEQ ID NO: 2 or an amino acid sequence with at least 80% or at least 90% identity thereto;
a heavy chain variable region of the amino acid sequence as set forth in any one of SEQ ID NOs: 31-35 or an amino acid sequence with at least 80% or at least 90% identity thereto, and a light chain variable region of the amino acid sequence as set forth in SEQ ID NO: 36 or an amino acid sequence with at least 80% or at least 90% identity thereto;
a heavy chain variable region of the amino acid sequence as set forth in any one of SEQ ID NOs: 31-35 or an amino acid sequence with at least 80% or at least 90% identity thereto, and a light chain variable region of the amino acid sequence as set forth in SEQ ID NO: 37 or an amino acid sequence with at least 80% or at least 90% identity thereto; or
a heavy chain variable region of the amino acid sequence as set forth in any one of SEQ ID NOs: 31-35 or an amino acid sequence with at least 80% or at least 90% identity thereto, and a light chain variable region of the amino acid sequence as set forth in SEQ ID NO: 38 or an amino acid sequence with at least 80% or at least 90% identity thereto.

In some embodiments, the BTN3A-binding protein comprises a heavy chain variable region of the amino acid sequence as set forth in any one of SEQ ID NOs: 3 and 39-44 or an amino acid sequence with at least 80% or at least 90% identity thereto, and a light chain variable region of the amino acid sequence as set forth in any one of SEQ ID NOs: 4 and 45-47 or an amino acid sequence with at least 80% or at least 90% identity thereto.

In some specific embodiments, the BTN3A-binding protein comprises:
a heavy chain variable region of the amino acid sequence as set forth in SEQ ID NO: 3 or an amino acid sequence with at least 80% or at least 90% identity thereto, and a light chain variable region of the amino acid sequence as set forth in SEQ ID NO: 4 or an amino acid sequence with at least 80% or at least 90% identity thereto;
a heavy chain variable region of the amino acid sequence as set forth in any one of SEQ ID NOs: 39-44 or an amino acid sequence with at least 80% or at least 90% identity thereto, and a light chain variable region of the amino acid sequence as set forth in SEQ ID NO: 45 or an amino acid sequence with at least 80% or at least 90% identity thereto;
a heavy chain variable region of the amino acid sequence as set forth in any one of SEQ ID NOs: 39-44 or an amino acid sequence with at least 80% or at least 90% identity thereto, and a light chain variable region of the amino acid sequence as set forth in SEQ ID NO: 46 or an amino acid sequence with at least 80% or at least 90% identity thereto; or
a heavy chain variable region of the amino acid sequence as set forth in any one of SEQ ID NOs: 39-44 or an amino acid sequence with at least 80% or at least 90% identity thereto, and a light chain variable region of the amino acid sequence as set forth in SEQ ID NO: 47 or an amino acid sequence with at least 80% or at least 90% identity thereto.

In some embodiments, the aforementioned BTN3A-binding protein is an anti-BTN3A antibody or an antigen-binding fragment thereof.

In some embodiments, the aforementioned antibody is a rabbit-derived antibody, a chimeric antibody, a humanized antibody, or a fully human antibody.

In some embodiments, the aforementioned antibody is a recombinant antibody.

In some embodiments, the aforementioned antibody is a monospecific antibody or a multispecific antibody (e.g., a bispecific antibody, a trispecific antibody, or a tetraspecific antibody).

In some embodiments, the aforementioned antibody or antigen-binding fragment thereof is a single-chain antibody (i.e., a full-length heavy chain and a full-length light chain), Fab, modified Fab, Fab', modified Fab', F(ab')₂, Fv, Fab-Fv, Fab-dsFv, a single-domain antibody (e.g., VH, VL, or VHH), scFv, a bivalent, trivalent, or tetravalent antibody, Bis-scFv, diabody, tribody, triabody, tetrabody, and an epitope-binding fragment of any one of the foregoing.

In some embodiments, the aforementioned antibody or antigen-binding fragment thereof is engineered by humanization, backmutation, affinity maturation, removal/reduction of a T cell epitope (TCE), reduction of antibody deamidation, and/or reduction of antibody isomerization.

In some embodiments, engineering by removal/reduction of a TCE results in one or more changes in one or more CDRs, which lead to reduced immunogenicity of the BTN3A-binding protein.

In some embodiments, in the human germline template used in the humanization engineering process, the heavy chain framework regions are derived from IGHV3-66, and the light chain framework regions are derived from IGkV1-27.

In some embodiments, the BTN3A-binding protein further comprises an Fc region of an immunoglobulin. In some specific embodiments, the Fc region is the Fc region of human IgG1, human IgG2, human IgG3, or human IgG4. In some specific embodiments, the Fc region may be an Fc region with reduced effector function, for example, the Fc region may have a mutation, and exemplary IgG Fc regions with reduced effector function include those with the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1), where "/" denotes "and". In some specific embodiments, the Fc region is the Fc region of human IgG1 with L234F, L235E, and/or P331S mutations, in which optionally, K446 is deleted. In some specific embodiments, the Fc region has the amino acid sequence of SEQ ID NO: 23 or 24 or an amino acid sequence with at least 80% or 90% identity thereto.

In some embodiments, the Fc region is more stable, or has a mutation that increases the stability of the Fc region compared to the wild-type Fc region. In some embodiments, the Fc region may allow the binding protein to form a dimeric molecule. In some embodiments, the Fc region may extend the *in vivo* half-life of the binding protein.

In some embodiments, the BTN3A-binding protein comprises a heavy chain of the amino acid sequence as set forth in any one of SEQ ID NOs: 25, 27, 48-52, and 56-61, or an amino acid sequence with at least 80% or at least 90% identity thereto, and/or a light chain of the amino acid sequence as set forth in any one of SEQ ID NOs: 26, 28, 53-55, and 62-64, or an amino acid sequence with at least 80% or at least 90% identity thereto.

In some specific embodiments, the BTN3A-binding protein comprises:
a heavy chain of the amino acid sequence as set forth in SEQ ID NO: 25 or an amino acid sequence with at least 80% or at least 90% identity thereto, and a light chain of the amino acid sequence as set forth in SEQ ID NO: 26 or an amino acid sequence with at least 80% or at least 90% identity thereto;
a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 53 or an amino acid sequence with at least 80% or at least 90% identity thereto;
a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 54 or an amino acid sequence with at least 80% or at least 90% identity thereto; or
a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 55 or an amino acid sequence with at least 80% or at least 90% identity thereto.

In some specific embodiments, the BTN3A-binding protein comprises:
a heavy chain of the amino acid sequence as set forth in SEQ ID NO: 27 or an amino acid sequence with at least 80% or at least 90% identity thereto, and a light chain of the amino acid sequence as set forth in SEQ ID NO: 28 or an amino acid sequence with at least 80% or at least 90% identity thereto;
a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 62 or an amino acid sequence with at least 80% or at least 90% identity thereto;
a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 63 or an amino acid sequence with at least 80% or at least 90% identity thereto; or
a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 64 or an amino acid sequence with at least 80% or at least 90% identity thereto.

In the present invention, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; and "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the BTN3A-binding protein specifically binds to one or more of BTN3A1, BTN3A2, and BTN3A3, for example,
it specifically binds to BTN3A1 alone, and does not specifically bind to BTN3A2 and BTN3A3;
it specifically binds to BTN3A2 alone, and does not specifically bind to BTN3A1 and BTN3A3;
it specifically binds to BTN3A3 alone, and does not specifically bind to BTN3A1 and BTN3A2;
it specifically binds to BTN3A1 and BTN3A2, and does not specifically bind to BTN3A3;
it specifically binds to BTN3A2 and BTN3A3, and does not specifically bind to BTN3A1;
it specifically binds to BTN3A1 and BTN3A3, and does not specifically bind to BTN3A2; or
it specifically binds to all of BTN3A1, BTN3A2, and BTN3A3.

In some specific embodiments, the above-mentioned BTN3A1, BTN3A2, and BTN3A3 are primate- or human-derived BTN3A1, BTN3A2, and BTN3A3.

In some embodiments, the BTN3A-binding protein of the present invention has one or more of the following properties:
(i) it binds to human BTN3A1 with an EC₅₀ of 0.1 nM, 0.05 nM, 0.02 nM, 0.01 nM, or lower, as measured by ELISA, which is a detection method well known in the art, e.g., as described in Part 1 of Example 3 of the present invention;
(ii) it binds to human BTN3A1 on cells with an EC₅₀ of 50 nM, 20 nM, 15 nM, 10 nM, 8 nM, 5 nM, or lower, as measured by FACS, which is a detection method well known in the art, e.g., as described in Part 2 of Example 3 of the present invention;
(iii) it binds to human BTN3A1 with a K_{D} of 5E-10M, 2E-10M, 1E-10M, or lower, as measured by Biacore, which is a detection method well known in the art, e.g., as described in Part 3 of Example 3 of the present invention;
(iv) it induces the activation and proliferation of γδ T cells (e.g., Vγ9Vδ2 T cells) with an EC₅₀ of 10 nM, 5 nM, 2 nM, 1 nM, or lower, as measured by a method well known in the art, e.g., as described in Part 4 of Example 3 of the present invention; and in some embodiments, the induction occurs upon co-culture of γδ T cells with cancer cells (e.g., SKOV-3 cells); and
(v) it induces γδ T cells (e.g., Vγ9Vδ2 T cells) to secrete cytokines (e.g., IFNγ); and in some embodiments, the induction occurs upon co-culture of γδ T cells with cancer cells (e.g., SKOV-3 cells).

The present invention also provides a BTN3A-binding protein, comprising any one or any combination of the following CDRs: HCDR1, HCDR2, and HCDR3 of the amino acid sequences as set forth in SEQ ID NOs: 17-19, respectively, and LCDR1, LCDR2, and LCDR3 of the amino acid sequences as set forth in SEQ ID NOs: 20-22, respectively.

In some embodiments, the present invention provides a variant of the aforementioned BTN3A-binding protein, which has one or more (e.g., 1, 2, 3, 4, 5, 6, 7, 8, 9, or 10) amino acid mutations compared to the heavy chain variable region of the amino acid sequence as set forth in any one of SEQ ID NOs: 1, 3, 31-35, and 39-44, and/or the light chain variable region of the amino acid sequence as set forth in any one of SEQ ID NOs: 2, 4, 36-38, and 45-47; wherein the amino acid mutations may be conservative replacements, substitutions, or modifications, and/or deletions or additions that do not affect function; and the amino acid mutations may occur in the CDR regions and/or FR regions.

In some embodiments, provided is a protein or molecule that binds to or competes for binding to the same epitope as the one to which the BTN3A-binding protein of the present invention as described above binds.

In some embodiments, provided is a protein or molecule that blocks the binding of the BTN3A-binding protein of the present invention as described above to BTN3A (including BTN3A1, BTN3A2, and/or BTN3A3).

In some embodiments, provided is a protein or molecule, the binding of which to BTN3A (including BTN3A1, BTN3A2, and/or BTN3A3) is blocked by the BTN3A-binding protein of the present invention as described above.

In some embodiments, provided is a protein or molecule, comprising a combination of any one or more of the heavy chain variable regions and light chain variable regions of the present invention as described above, wherein the heavy chain variable regions are selected from SEQ ID NOs: 1, 3, 31-35, and 39-44, and the light chain variable regions are selected from SEQ ID NOs: 2, 4, 36-38, and 45-47. For example, the protein or molecule is a conjugate, which may, for example, comprise any detectable label.

### Polynucleotide and vector

The present invention provides a polynucleotide encoding the BTN3A-binding protein of the present invention. The nucleic acid of the present invention may be RNA or DNA (e.g., cDNA). According to some embodiments of the present invention, the nucleic acid of the present invention is a substantially isolated nucleic acid.

The nucleic acid of the present invention may also be in the form of a vector, may be present in a vector, and/or may be part of a vector, such as a plasmid, a cosmid, YAC, or a viral vector. The vector may in particular be an expression vector, i.e., a vector that enables the expression of the BTN3A-binding protein *in vitro* and/or *in vivo* (i.e., in a suitable host cell, host organism, and/or expression system). The expression vector generally comprises at least one nucleic acid of the present invention operably linked to one or more suitable expression regulatory elements (e.g., promoters, enhancers, terminators, etc.). It is common knowledge to those skilled in the art to select the elements and their sequences for expression in a particular host. Regulatory elements and other elements useful or necessary for the expression of the BTN3A-binding protein of the present invention include, for example, promoters, enhancers, terminators, integration factors, selectable markers, leader sequences, and reporter genes.

The nucleic acid of the present invention may be prepared or obtained by known methods (e.g., by automatic DNA synthesis and/or recombinant DNA technology) based on the information of the amino acid sequence of the polypeptide of the present invention, and/or may be isolated from a suitable natural source.

### Host cell

The present invention provides a recombinant host cell that expresses or is capable of expressing one or more BTN3A-binding proteins of the present invention and/or comprises the polynucleotide or vector of the present invention. In some embodiments, the host cell is a bacterial cell, a fungal cell, or a mammalian cell.

Bacterial cells include, for example, cells from Gram-negative bacterial strains (e.g., *Escherichia coli* strains, *Proteus* strains, and *Pseudomonas* strains) and Grampositive bacterial strains (e.g., *Bacillus* strains, *Streptomyces* strains, *Staphylococcus* strains, and *Lactococcus* strains).

Fungal cells include, for example, cells of species of *Trichoderma, Neurospora,* and *Aspergillus*; or cells of species of *Saccharomyces* (e.g., *Saccharomyces cerevisiae), Schizosaccharomyces* (e.g., *Schizosaccharomyces pombe), Pichia* (e.g., *Pichia pastoris* and *Pichia methanolica),* and *Hansenula.*

Mammalian cells include, for example, HEK293 cells, CHO cells, BHK cells, HeLa cells, COS cells, etc.

However, amphibian cells, insect cells, plant cells, and any other cells used in the art to express heterologous proteins may also be used in the present invention.

### Preparation method

The present invention provides a method for preparing a BTN3A-binding protein, comprising: expressing the protein of interest in the host cell as previously described, and isolating the protein of interest from the host cell. Optionally, the method may further comprise a purification step, for example, purifying the isolated protein with an A or G Sepharose FF column containing buffer with adjusted pH, washing away non-specifically bound components, then eluting the bound antibody using a pH gradient method, detecting the eluted antibody by SDS-PAGE, and collecting the antibody. Optionally, filtration and concentration are performed by conventional methods. Soluble aggregates and multimers may also be removed by conventional methods, such as molecular sieving and ion exchange. The resulting product must be immediately frozen (e.g., at -70°C) or lyophilized.

Methods for producing and purifying antibodies are well known and available in the prior art, for example, in Antibodies: A Laboratory Manual, Cold Spring Harbor Laboratory Press (Chapters 5-8 and 15).

The binding protein of the present invention (e.g., an engineered antibody or antigen-binding fragment) may be prepared and purified by conventional methods. For example, the cDNA sequences encoding the heavy chain and light chain may be cloned and recombined into an expression vector. The recombinant immunoglobulin expression vector may be stably transfected into CHO cells. Mammalian expression systems result in glycosylation of antibodies, especially at the highly conserved N-terminus of the Fc region. Stable clones are obtained by expressing antibodies that specifically bind to the antigen. Positive clones are expanded in a serum-free medium in a bioreactor to produce antibodies. The culture medium containing the secreted antibodies may be purified and collected by conventional techniques. The antibodies may be filtered and concentrated by conventional methods. Soluble aggregates and multimers may also be removed by conventional methods, such as molecular sieving and ion exchange.

### Composition

The present invention provides a composition, comprising the BTN3A-binding protein of the present invention as described above. For example, provided is a pharmaceutical composition, comprising an amount of the aforementioned BTN3A-binding protein effective to treat, alleviate, or prevent a disease, and at least one pharmaceutically acceptable excipient, diluent, or carrier.

In some specific embodiments, the pharmaceutical composition may comprise from 0.01 to 99% by weight of the BTN3A-binding protein in a unit dose, or the amount of the BTN3A-binding protein in a unit dose of the pharmaceutical composition is from 0.1 to 2000 mg, and in some specific embodiments, from 1 to 1000 mg.

In some embodiments, provided is an article of manufacture or product (e.g., a kit), comprising the aforementioned BTN3A-binding protein. Optionally, the article of manufacture comprises a container and a label. The container is, for example, a vial, a syringe, or a test tube. The container contains a composition effective for treating a condition. The label on or attached to the container indicates that the composition is used to treat the selected condition.

In some embodiments, the aforementioned disease is cancer.

### Method of treatment and pharmaceutical use

The present invention provides a method for treating, alleviating, preventing, or diagnosing a disease or condition using the aforementioned BTN3A-binding protein, polynucleotide, or composition (including pharmaceutical composition).

In some embodiments, provided is a method for improving, alleviating, treating, or preventing a disease, comprising administering an amount of the aforementioned BTN3A-binding protein, polynucleotide, or composition (including pharmaceutical composition) effective for improving, alleviating, treating, or preventing the disease to a subject in need thereof.

In some embodiments, provided is a use of the BTN3A-binding protein, polynucleotide, and composition (including pharmaceutical composition) of the present invention in the preparation of a medicament for improving, alleviating, treating, or preventing a disease.

In some embodiments, the aforementioned disease is a disease or condition associated with overexpression of BTN3A.

In some embodiments, the aforementioned disease is a disease or condition mediated by BTN3A.

In some embodiments, the aforementioned disease is cancer.

In some embodiments, provided is a method for activating γδ T cells and promoting cytokine release by γδ T cells, comprising administering to the γδ T cells or contacting the γδ T cells with the BTN3A-binding protein, polynucleotide, or composition (including pharmaceutical composition), wherein the γδ T cells may be *in vivo* in a subject in need thereof or *ex vivo* from the subject. In some specific embodiments, the γδ T cells are Vγ9Vδ2 T cells.

### Detection

The present invention provides a detection use of the BTN3A-binding protein, polynucleotide, and composition. The present invention also provides a method, a system, or a device for detecting BTN3A *in vivo* or *in vitro,* which comprises treating a sample with the aforementioned binding protein, polynucleotide, or composition of the present invention.

In some embodiments, an *in vitro* detection method, system, or device may, for example, comprise:
(1) contacting a sample with the BTN3A-binding protein, polynucleotide, or composition of the present invention;
(2) detecting a complex formed between the aforementioned binding protein or polynucleotide and the sample; and/or
(3) contacting a reference sample (e.g., a control sample) with the binding protein or nucleic acid; and
(4) determining the degree of formation of the complex by comparing with the reference sample. A change in complex formation (e.g., a statistically significant change) in a sample as compared to a control sample indicates the presence of BTN3A in the sample.

In certain embodiments, also provided is a kit, which comprises the aforementioned BTN3A-binding protein or polynucleotide, and may further comprise diagnostic instructions for use. The kit may also comprise at least one additional reagent, such as a marker or additional diagnostic agent. For *in vivo* use, the BTN3A-binding protein may be formulated as a pharmaceutical composition.

### Term definition

In order that the present invention can be more readily understood, certain technical and scientific terms are specifically defined below. Unless expressly defined otherwise in the present invention, all other technical and scientific terms used in the present invention have the meanings commonly understood by one of ordinary skill in the art to which the present invention belongs.

The three-letter and single-letter codes for amino acids used in the present invention are described in J. biol. chem, 243, p 3558 (1968).

"BTN3A" has its ordinary meaning in the art, and refers to the human BTN3A polypeptide, exemplarily BTN3A1 as set forth in Genbank Accession NO. NP_008979.3, BTN3A2 as set forth in Genbank Accession NO. NP_008978.2, or BTN3A3 as set forth in Genbank Accession NO. NP_008925.1.

"BTN3A-binding protein" encompasses any protein capable of specifically binding to BTN3A or any molecule comprising the protein, including but not limited to anti-BTN3A antibodies as defined in the present invention and directed against BTN3A, antigen-binding fragments thereof, or conjugates thereof. In some embodiments, the BTN3A-binding protein may comprise a linker, and/or a moiety with effector function such as a half-life extending moiety (e.g., an immunoglobulin single variable domain that binds to serum albumin), and/or a fusion partner (e.g., serum albumin), and/or a conjugated polymer (e.g., PEG), and/or an Fc region.

"Antibody" encompasses various antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding portions), so long as they exhibit the desired antigen-binding activity. An antibody may refer to an immunoglobulin, which is a tetrapeptide chain structure composed of two heavy chains and two light chains linked via interchain disulfide bonds. The immunoglobulin heavy chain constant regions have different amino acid compositions and sequence arrangements, and therefore have different antigenicity. Accordingly, immunoglobulins can be classified into five classes, or referred to as the isotypes of immunoglobulins, namely IgM, IgD, IgG, IgA, and IgE, whose corresponding heavy chains are the µ chain, δ chain, γ chain, α chain, and ε chain, respectively. Ig of the same class can be further classified into different subclasses based on differences in the amino acid composition of the hinge regions and the number and positions of the heavy chain disulfide bonds, for example, IgG can be divided into IgG1, IgG2, IgG3, and IgG4. Light chains are classified into κ chains or λ chains based on differences in the constant regions. Each of the five classes of Ig can have κ chains or λ chains. In the antibody heavy chains and light chains, the sequences of about 110 amino acids near the N-termini vary greatly, and are referred to as the variable regions (V regions); and the remaining amino acid sequences near the C-termini are relatively stable, and are referred to as the constant regions (C regions). The variable region comprises three hypervariable regions (HVRs) and four framework regions (FRs) with relatively conserved sequences. The three hypervariable regions determine the specificity of the antibody, and are also referred to as complementarity determining regions (CDRs). Each light chain variable region (VL) and heavy chain variable region (VH) consists of three CDR regions and four FR regions, arranged in the order from the amino-terminus to the carboxy-terminus: FR1, CDR1, FR2, CDR2, FR3, CDR3, and FR4. The three CDR regions of the light chain refer to LCDR1, LCDR2, and LCDR3; and the three CDR regions of the heavy chain refer to HCDR1, HCDR2, and HCDR3.

Antibodies of the present invention can be polyclonal, monoclonal, xenogeneic, allogeneic, syngeneic, or modified forms thereof, among which monoclonal antibodies are particularly suitable in various embodiments. In general, the antibodies of the present invention are recombinant antibodies. "Recombinant" as used herein generally refers to products such as cells, nucleic acids, proteins, or vectors, meaning that the cells, nucleic acids, proteins, or vectors have been modified by the introduction of heterologous nucleic acids or proteins or by the alteration of native nucleic acids or proteins, or that the cells are derived from cells so modified. For example, recombinant cells express genes that are not found within the native (non-recombinant) form of the cell, or express native genes that are otherwise abnormally expressed, under expressed, or not expressed at all.

"Antigen-binding fragment" encompasses single-chain antibodies (i.e., full-length heavy chains and full-length light chains), Fab, modified Fab, Fab', modified Fab', F(ab')₂, Fv, Fab-Fv, Fab-dsFv, single-domain antibodies (e.g., VH, VL, or VHH), scFv, bivalent, trivalent, or tetravalent antibodies, Bis-scFv, diabody, tribody, triabody, tetrabody, and epitope-binding fragments of any one of the foregoing (see, e.g., Holliger and Hudson, 2005, Nature Biotech. 23(9): 1126-1136; and Adair and Lawson, 2005, Drug Design Reviews-Online 2(3), 209-217). Methods for producing and preparing these antigen-binding fragments are well known in the art (see, e.g., Verma et al., 1998, Journal of Immunological Methods, 216, 165-181).

For the determination or definition of CDRs, definitive delineation of CDRs and identification of residues comprising the binding site of an antibody can be accomplished by resolving the structure of the antibody and/or resolving the structure of the antibody-ligand complex. This can be achieved by any of a variety of techniques known to those skilled in the art, such as X-ray crystallography. A variety of analytical methods can be used to identify CDRs, including but not limited to the Kabat numbering system, the Chothia numbering system, the AbM numbering system, the IMGT numbering system, contact definition, and conformational definition. The Kabat numbering system is a standard for numbering residues in antibodies, and is commonly used to identify CDR regions (see, e.g., Johnson & Wu, 2000, Nucleic Acids Res., 28: 214-8). The Chothia numbering system is similar to the Kabat numbering system, but the Chothia numbering system contemplates the positions of certain structural loop regions (see, e.g., Chothia et al., 1986, J. Mol. Biol., 196: 901-17; and Chothia et al., 1989, Nature, 342: 877-83). The AbM numbering system uses an integrated suite of computer programs produced by Oxford Molecular Group that models antibody structures (see, e.g., Martin et al., 1989, Proc Natl Acad Sci (USA), 86: 9268-9272; and "AbMTM, A Computer Program for Modeling Variable Regions of Antibodies", Oxford, UK; Oxford Molecular, Ltd). The AbM numbering system uses a combination of knowledge databases and *de novo* methods to model the tertiary structure of antibodies from the primary sequence (see those described by Samudrala et al., 1999, in "Ab Initio Protein Structure Prediction Using a Combined Hierarchical Approach" in PROTEINS, Structure, Function and Genetics Suppl., 3: 194-198). The contact definition is based on the analysis of available complex crystal structures (see, e.g., MacCallum et al., 1996, J. Mol. Biol., 5: 732-45). In the conformational definition, the positions of CDRs can be identified as residues that make enthalpic contributions to antigen binding (see, e.g., Makabe et al., 2008, Journal of Biological Chemistry, 283: 1156-1166). Additionally, other CDR boundary definitions may not strictly adhere to one of the above-mentioned methods but still overlap with at least a portion of the Kabat CDRs, even though they may be shortened or lengthened based on the fact that specific residues or groups of residues do not significantly affect antigen binding (as confirmed by predictive or experimental results). As used in the present invention, a CDR may refer to a CDR defined by any method known in the art, including combinations of such methods. The correspondences among various numbering systems are well known to those skilled in the art.

"Domain" of a polypeptide or protein refers to a folded protein structure which has the ability to retain its tertiary structure independently of the rest of the protein. Generally, domains are responsible for individual functional properties of proteins, and in many cases may be added, removed, or transferred to other proteins without loss of function of the remainder of the protein and/or of the domain.

"Antibody framework (FR)" refers to a portion of a variable domain that serves as a scaffold for the antigen-binding loops (CDRs) of the variable domain.

"Humanized antibody", also known as CDR-grafted antibody, refers to an antibody produced by grafting non-human CDR sequences into the framework of human antibody variable regions. Humanized antibodies can overcome the strong immune response induced by chimeric antibodies due to their high content of non-human protein components. In order to avoid a reduction in activity concomitant with the decrease in immunogenicity, minimum reverse mutations may be introduced into the fully human antibody variable regions to maintain activity. Examples of "humanization" include the "humanization" of Camelidae-derived VHH domains by replacing one or more amino acid residues in the amino acid sequence of the original VHH sequence with one or more amino acid residues present at the corresponding positions in the VH domain of human conventional antibodies with a tetrapeptide chain structure. The humanized VHH domain may comprise one or more fully human framework region sequences, and in some specific embodiments, the human framework region sequences of IGHV3. Humanization methods include, for example, protein surface amino acid humanization (resurfacing) and universal framework grafting method for antibody humanization (CDR grafting to a universal framework), i.e., "grafting" CDRs onto other "scaffolds" (including but not limited to human scaffolds or nonimmunoglobulin scaffolds). Scaffolds and techniques suitable for such CDR grafting are known in the art. For example, germline DNA sequences of human heavy and light chain variable region genes can be found in the VBase human germline sequence database and in Kabat, E. A. et al., 1991 Sequences of Proteins of Immunological Interest, 5th ed. The humanized antibody of the present invention also includes humanized antibodies which are further subjected to CDR affinity maturation by phage display. In addition, in order to avoid a reduction in activity concomitant with the decrease in immunogenicity, minimum reverse mutations or backmutations may be introduced into the human antibody variable region framework sequences to maintain activity.

"Affinity-matured" antibody refers to an antibody that has one or more alterations in one or more hypervariable regions (HVRs) as compared to a parental antibody lacking such alterations, wherein such alterations result in improved affinity of the antibody for the antigen. For example, an "affinity-matured" BTN3A-binding protein or anti-BTN3A antibody has one or more alterations in one or more CDRs, which result in increased affinity for the antigen as compared to its parental antibody. Affinity-matured antibodies can be prepared, for example, by methods known in the art as described below: Marks et al., 1992, Biotechnology 10: 779-783; Barbas et al., 1994, Proc. Nat. Acad. Sci, USA 91: 3809-3813.; Shier et al., 1995, Gene 169: 147-155; Yelton et al., 1995, Immunol. 155: 1994-2004; Jackson et al., 1995, J. Immunol. 154(7): 3310-9; Hawkins et al., 1992, J. MoI. Biol. 226(3): 889896; and KS Johnson and RE Hawkins, "Affinity maturation of antibodies using phage display", Oxford University Press 1996.

Typically, the BTN3A-binding protein of the present invention will bind the antigen or target protein to be bound (i.e., BTN3A1, BTN3A2, or BTN3A3) with a dissociation constant (K_{D}) of preferably 10⁻⁷ to 10⁻¹⁰ moles per liter (M), more preferably 10⁻⁸ to 10⁻¹⁰ M, even more preferably 10⁻⁹ to 10⁻¹⁰ M, or lower, and/or an association constant (KA) of at least 10⁻⁷ M, preferably at least 10⁻⁸ M, more preferably at least 10⁻⁹ M, and even more preferably at least 10⁻¹⁰ M, as measured in a Biacore, KinExA, or Fortibio assay. Any K_{D} value greater than 10⁻⁴ M is generally considered to indicate non-specific binding. Specific binding of an antigen-binding protein to an antigen or epitope can be determined in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) as described in the present invention.

"Binding affinity" or "affinity" is used in the present invention as a measure of the strength of the non-covalent interaction between two molecules (e.g., an antibody or a portion thereof and an antigen). The binding affinity between two molecules can be quantified by determining the dissociation constant (K_{D}). K_{D} can be determined by measuring the kinetics of complex formation and dissociation by using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be directly determined by well-known methods, and can even be calculated for complex mixtures by, for example, those methods described in Caceci et al. (1984, Byte 9: 340-362). For example, K_{D} can be determined by using a dual filtration nitrocellulose filter binding assay such as that disclosed in Wong & Lohman (1993, Proc. Natl. Acad. Sci. USA 90: 5428-5432). Other standard assays for evaluating the binding ability of an antibody to a target antigen are known in the art, and include, for example, ELISA, Western blotting, RIA, and flow cytometry, as well as other assays exemplified elsewhere in the present invention. The binding kinetics and binding affinity of an antibody can also be evaluated by standard assays known in the art, such as surface plasmon resonance (SPR), for example, by using the Biacore^{™} system or KinExA. The binding affinities associated with different molecular interactions, e.g., the binding affinities of different antibodies for a given antigen, can be compared by comparing the K_{D} values of various antibody/antigen complexes. Similarly, the specificity of an interaction can be evaluated by determining and comparing the K_{D} value for the interaction of interest (e.g., the specific interaction between an antibody and an antigen) with the K_{D} value for an interaction not of interest (e.g., the interaction between a control antibody known not to bind to BTN3A and the antigen).

"Conservative substitution" refers to a substitution with another amino acid residue having similar properties to the original amino acid residue. For example, lysine, arginine, and histidine have similar properties in that they have basic side chains, and aspartic acid and glutamic acid have similar properties in that they have acidic side chains. Furthermore, glycine, asparagine, glutamine, serine, threonine, tyrosine, cysteine, and tryptophan have similar properties in that they have uncharged polar side chains, and alanine, valine, leucine, isoleucine, proline, phenylalanine, and methionine have similar properties in that they have non-polar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in both of the two compared sequences are occupied by identical nucleotides or amino acid monomers, e.g., if a position of each of two DNA molecules is occupied by an identical nucleotide, the molecules are homologous at that position. The percent homology between two sequences is a function of the number of matched or homologous positions shared by the two sequences divided by the number of positions compared × 100%. For example, if 6 out of 10 positions are matched or homologous in two sequences when the sequences are optimally aligned, the two sequences are 60% homologous. Generally, a comparison is made when two sequences are aligned to give maximum percent homology.

"Nucleic acid" and "polynucleotide" are used interchangeably in the present invention to refer to any DNA or RNA molecule, either single- or double-stranded, and in the case of a single-stranded molecule, a molecule of its complementary sequence, preferably a double-stranded DNA. A nucleic acid is "operably linked" when it is placed into a functional relationship with another nucleic acid sequence. For instance, a promoter or enhancer is operably linked to a coding sequence if the promoter or enhancer affects the transcription of the coding sequence.

"Host cell" includes individual cells or cell cultures, which may be or have been the recipient of a vector for incorporation of a polynucleotide insert. A host cell includes progeny of a single host cell, and the progeny may not necessarily be completely identical (in morphology or in genomic DNA complement) to the original parent cell due to natural, accidental, or deliberate mutation. A host cell includes cells transfected and/or transformed *in vivo* with a polynucleotide of the present invention. "Cell", "cell line", and "cell culture" are used interchangeably, and all such designations include their progeny. It is also understood that all progeny may not be precisely identical in DNA content, due to deliberate or inadvertent mutations. Variant progeny that have the same function or biological activity as screened for in the originally transformed cell are included.

"Inhibition" and "blockade" are used interchangeably, and encompass both partial and complete inhibition/blockade. "Inhibition of growth" (e.g., involving cells) is intended to include any measurable reduction in cell growth.

"Giving", "administering", and "treating", when applied to animals, humans, experimental subjects, cells, tissues, organs, or biological fluids, refer to contact of an exogenous drug, therapeutic agent, diagnostic agent, or composition with the animals, humans, subjects, cells, tissues, organs, or biological fluids, for example in therapeutic, pharmacokinetic, diagnostic, research, and experimental methods. Treatment of cells includes contacting the reagent with the cells and contacting the reagent with a fluid, wherein the fluid is in contact with the cells. "Giving", "administering", and "treating" also means treating, e.g., cells, with a reagent, a diagnostic agent, a binding composition, or with another cell *in vitro* and *ex vivo.* When applied to humans, veterinary, or research subjects, they refer to therapeutic treatment, preventive or prophylactic measures, and research and diagnostic applications.

"Treating" means administering a therapeutic agent, such as a therapeutic agent comprising any binding protein of the present invention or a pharmaceutical composition thereof, either internally or externally, to a subject who has had, is suspected of having, or is predisposed to having one or more proliferative diseases or symptoms thereof, on which the therapeutic agent is known to have a therapeutic effect. Typically, the therapeutic agent is administered in an amount effective to alleviate one or more disease symptoms in the subject or population being treated, whether by inducing regression of such symptoms or inhibiting the development of such symptoms into any clinically measurable degree. The amount of therapeutic agent effective to alleviate any particular disease symptom (also referred to as the "therapeutically effective amount") may vary depending on various factors such as the disease state, age, and weight of the subject, and the ability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present invention (e.g., treatment methods or articles of manufacture) may be ineffective in alleviating disease symptoms of interest in a certain subject, they shall alleviate the disease symptoms of interest in a statistically significant number of subjects, as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" includes an amount sufficient to ameliorate or prevent a symptom or sign of a medical condition. An effective amount also means an amount sufficient to allow or facilitate diagnosis. The effective amount for a subject may vary depending on the factors such as the condition to be treated, the general health of the subject, the route and dose of administration, and the severity of side effects. An effective amount can be the maximum dose or administration regimen that avoids significant side effects or toxic effects.

"Optional" or "optionally" means that the subsequently described event or circumstance may but need not occur, and that the description includes instances where the event or circumstance occurs and instances in which it does not. "And/or" should be taken as specific disclosure of each of the two specified features or components with or without the other. Thus, the term "and/or" as used in a phrase such as "A and/or B" in the present invention includes "A and B", "A or B", "A" (alone), and "B" (alone). Unless the context clearly requires otherwise, throughout the specification and the claims, the words "comprising", "having", "including", and the like are to be understood in an inclusive sense, rather than an exclusive or exhaustive sense; that is to say, in the sense of "including but not limited to". In the context of mutations comprised in the Fc region of the present invention, "/" denotes "and", for example, "L234A/L235A" denotes "L234A and L235A", that is, the Fc region comprises L234A and L235A mutations; and all the mutated amino acid positions in the Fc region of the present invention are defined in accordance with the EU numbering system.

"Subject" or "patient" in the present invention means a mammal, particularly a primate, and especially a human.

### Brief Description of the Drawings

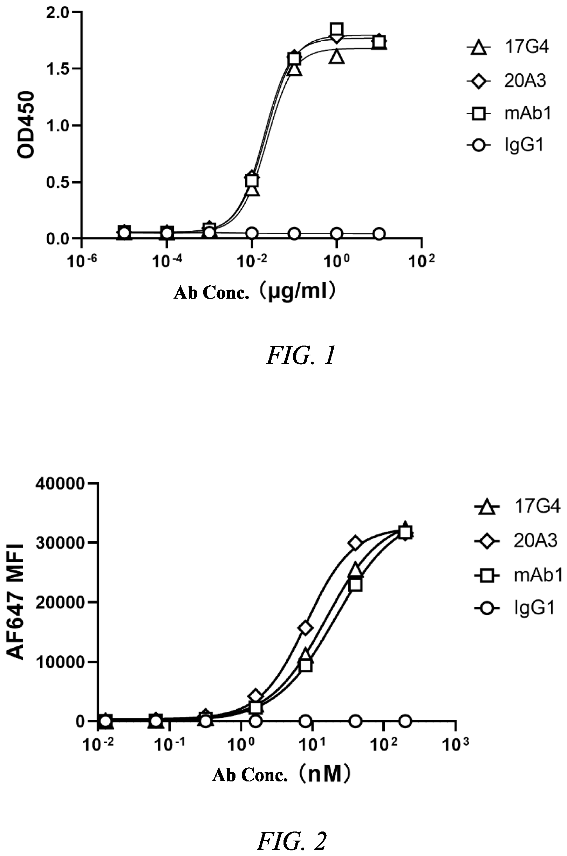

Figure 1 shows the results of the ELISA assay for the binding ability of the anti-BTN3A1 chimeric antibodies to the human BTN3A1 recombinant protein.
Figure 2 shows the results of the FACS assay for the binding ability of the anti-BTN3A1 chimeric antibodies to human BTN3A1-overexpressing cells.

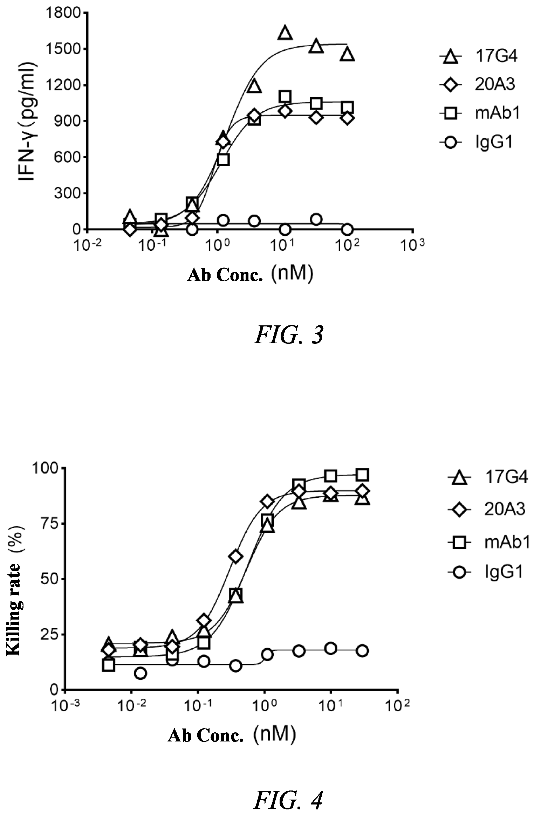

Figure 3 shows the results of the assay for the activation capacity of the anti-BTN3A1 chimeric antibodies on γδ T cells. Vγ9Vδ2 T cells are co-cultured with A375 target cells, the antibody to be tested is added and the mixture is incubated, and the amount of human IFNγ secreted in the cell supernatant is quantified by ELISA.
Figure 4 shows the results of the assay for the killing ability of γδ T cells against A375 cells mediated by the anti-BTN3A1 chimeric antibodies. Vγ9Vδ2 T cells are co-cultured with A375 cells, the antibody to be tested is added and the mixture is incubated, and the luminescent activity is measured using a microplate reader after the cells are lysed with a reagent.

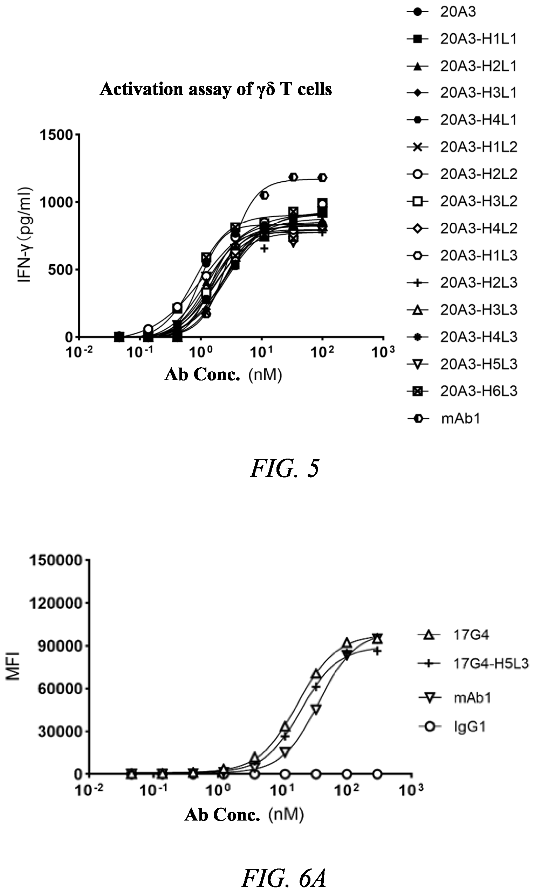

Figure 5 shows the results of the assay for the activation capacity of 20A3's humanized antibodies on γδ T cells. Vγ9Vδ2 T cells are co-cultured with A375 target cells, the antibody to be tested is added and the mixture is incubated, and the amount of human IFNγ secreted in the cell supernatant is quantified by ELISA.

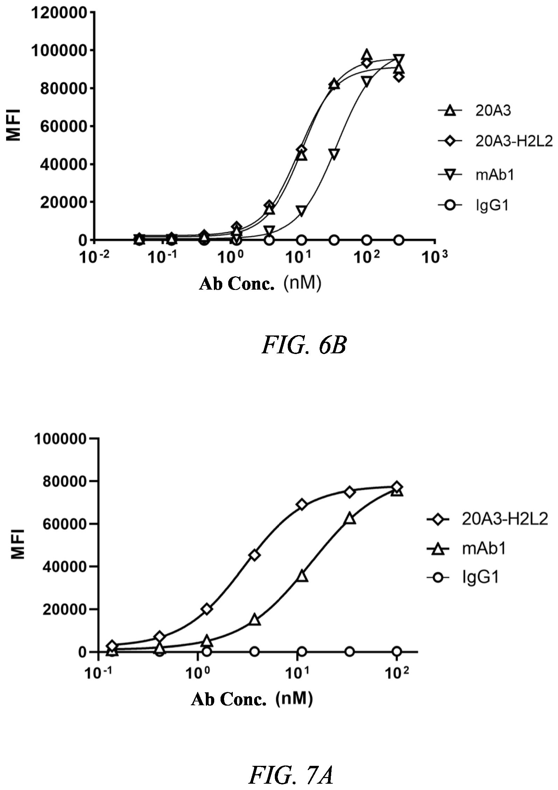

Figure 6A shows the results of the FACS assay for the binding ability of the 17G4's humanized antibody to human BTN3A1-overexpressing cells, and Figure 6B shows the results of the FACS assay for the binding ability of the 20A3's humanized antibody to human BTN3A1-overexpressing cells.

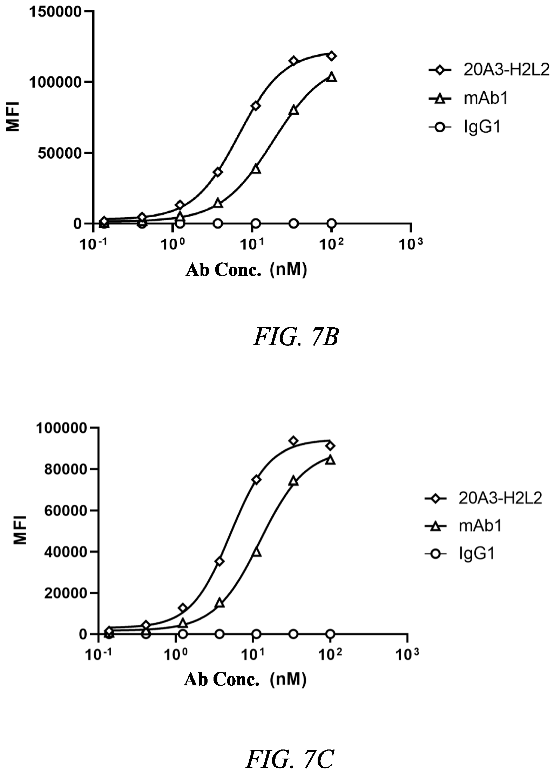

Figure 7A shows the results of the FACS assay for the binding ability of 20A3-H2L2 to human BTN3A1-overexpressing cells, Figure 7B shows the results of the FACS assay for the binding ability of 20A3-H2L2 to human BTN3A2-overexpressing cells, and Figure 7C shows the results of the FACS assay for the binding ability of 20A3-H2L2 to human BTN3A3-overexpressing cells.

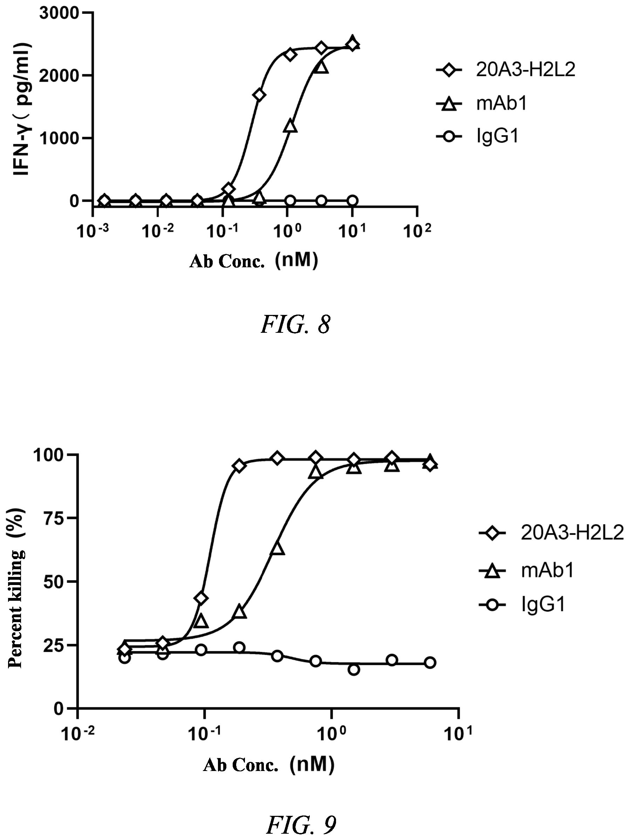

Figure 8 shows the results of the assay for the activation capacity of 20A3-H2L2 on γδ T cells. Vγ9Vδ2 T cells are co-cultured with SKOV-3 target cells, the antibody to be tested is added and the mixture is incubated, and the amount of human IFNγ secreted in the cell supernatant is quantified by ELISA.
Figure 9 shows the results of the assay for the killing ability of γδ T cells against SKOV-3 cells mediated by 20A3-H2L2. Vγ9Vδ2 T cells are co-cultured with SKOV-3 cells, the antibody to be tested is added and the mixture is incubated, and the luminescent activity is measured using a microplate reader after the cells are lysed with a reagent.

### Detailed Description of Embodiments

The present invention is further described below with reference to examples, but these examples are not intended to limit the scope of the present invention. In the examples of the present invention, experimental methods for which specific conditions are not specified are generally performed under conventional conditions, such as those described in Antibodies: A Laboratory Manual and Molecular Cloning: A Laboratory Manual, Cold Spring Harbor Laboratory Press; or in accordance with the conditions recommended by the manufacturers of the raw materials or commercial products. Reagents for which the specific source is not specified are conventional commercially available reagents.

### Example 1. Screening and preparation of anti-human BTN3A1 rabbit-derived monoclonal antibodies

### 1. Immunizing antigens and screening antigens

The human BTN3A1 recombinant protein with mouse IgG2a Fc and his tag (h-BTN3A1-mFc-his), the biotinylated human BTN3A1 recombinant protein with his and avi tags (Bio-h-BTN3A1-his-avi), and the human BTN3A1 recombinant protein with his tag (h-BTN3A1-his) are all commercially available protein reagents, and the sources of their respective sequences are shown in Table 1. The protein reagents can be used in each of the experiments described below.

**Table 1. Sources of recombinant protein amino acid sequences**

| Name | Start and end of amino acid sequence | Genbank Accession NO. | Company |
|---|---|---|---|
| h-BTN3A1-mFc-his | | | DIMA biotech |
| Bio-h-BTN3A1-his-avi | Gln30-Gly254 | 000481-1 | |
| h-BTN3A1-his | | | Kactus |

### 2. Screening of anti-human BTN3A1 rabbit-derived monoclonal antibodies

Antibodies were produced by immunizing New Zealand White rabbits.

Experimental method: The immunizing antigen was human BTN3A1 recombinant protein with mouse IgG2a Fc and his tag (h-BTN3A1-mFc-his). For the primary immunization, the rabbits were immunized with the antigen emulsified in an equal volume of Complete Freund's Adjuvant (CFA) at a dose of 300 µg per rabbit; and for the subsequent booster immunizations, the rabbits were immunized with the antigen emulsified in an equal volume of Incomplete Freund's Adjuvant (IFA) at a dose of 150 µg per rabbit. The immunizations were administered on Days 0, 22, 36, and 50. On Day 42, blood was collected for blood testing, and the sera from New Zealand White rabbits were tested by ELISA to determine the antibody titer in the rabbit sera. Spleens were harvested from rabbits with high antibody titers as determined by ELISA, and splenocytes were isolated. B cells that bound to the biotinylated human BTN3A1 recombinant protein (Bio-h-BTN3A1-his-avi) were sorted by flow cytometry, and plated in 96-well plates at a density of less than one B cell per well. After two weeks of culture, the B cell supernatants were collected.

### 3. ELISA binding assay of anti-BTN3A1 rabbit-derived monoclonal antibodies to BTN3A1 recombinant protein

The binding properties of rabbit-derived B cell supernatants to human BTN3A1 were determined by ELISA.

Experimental method: 96-well ELISA plates were coated with the human BTN3A1 recombinant protein with his tag (h-BTN3A1-his). After rabbit-derived monoclonal antibody supernatants were added, the binding activity between the antibody and the antigen was measured by adding a secondary antibody (HRP-conjugated anti-primary antibody Fc antibody) and the HRP substrate TMB. 96-well ELISA plates were coated with the human BTN3A1 recombinant protein with his tag (h-BTN3A1-his) at a concentration of 1 µg/mL, and incubated overnight at 4°C. The coated plates were spun dry and patted dry on absorbent paper, 150 µL of blocking buffer was added per well, and the ELISA plates were incubated for 1 hour at 37°C. The plates were spun dry and patted dry on absorbent paper. The anti-BTN3A1 antibodies to be tested were added to each well, and the plates were incubated for 1 hour at 37°C. The supernatants were discarded, and the plates were patted dry. 100 µL of HRP-labeled goat anti-rabbit IgG secondary antibody (diluted 1 : 5000 in dilution buffer) was added per well, and the plates were incubated for 1 hour at 37°C. The ELISA plates were washed with a plate washer using PBS, with a program of 5 washing cycles and a 30-second soak per cycle. Subsequently, 100 µL of substrate solution (TMB) was added per well, and the mixtures were reacted for 5 minutes to allow color development. The reactions were finally terminated by adding 100 µL of 1 mol/L sulfuric acid per well. The OD450 value was measured using a microplate reader (Tecan-infinite F50) at a wavelength of 450 nm.

We found that two rabbit-derived monoclonal antibodies exhibited higher binding activity to the human BTN3A1 recombinant protein, r17G4 and r20A3, respectively.

**Table 2. Results of ELISA binding assay of anti-BTN3A1 rabbit-derived monoclonal antibodies to human BTN3A1 recombinant protein**

| Sample | ELISA (OD 450) |
|---|---|
| Antibody r17G4 | 1.8404 |
| Antibody r20A3 | 2.1022 |
| Rabbit polyclonal antiserum (positive control) | 2.3977 |
| Blank (negative control) | 0.0539 |

### 4. Binding assay of anti-BTN3A1 rabbit-derived monoclonal antibodies to BTN3A1-expressing cells

The binding properties of rabbit-derived B cell supernatants to HEK293 cells expressing the human BTN3A1 protein were determined by flow cytometry (FACS).

Experimental method: A cell line transiently expressing human BTN3A1 was constructed. After the addition of antibodies, the binding properties of the antibodies to the antigen were determined by adding a secondary antibody. Expression plasmids containing the human BTN3A1 gene sequence were transiently transfected into HEK293 cells. Subsequently, 2 × 10⁵ transiently transfected HEK293 cells were seeded per well in 96-well plates. The plates were centrifuged for 5 minutes at 300 g, and the supernatants were discarded. 100 µL of antibodies to be tested were added per well, and the plates were incubated for 1 hour at 4°C. The supernatants were discarded by centrifugation, and the plates were washed three times with 200 µL of washing buffer (PBS + 2% FBS). Then, 100 µL of anti-rabbit IgG secondary antibody (diluted 1 : 500) was added, and the plates were incubated for 1 hour at 4°C. The supernatants were discarded by centrifugation, and the plates were washed three times with 200 µL of washing buffer (PBS + 2% FBS). Finally, the cells were resuspended in 100 µL of PBS and analyzed by flow cytometry.

The results showed that r17G4 and r20A3 were able to bind significantly to BTN3A1-expressing HEK293 cells.

**Table 3. Results of binding assay of anti-BTN3A1 rabbit-derived monoclonal antibodies to BTN3A1-expressing HEK293 cells**

| Sample | MFI | Percentage (%) |
|---|---|---|
| Antibody r17G4 | 443884 | 99 |
| Antibody r20A3 | 189571 | 98.33 |
| Rabbit polyclonal antiserum (positive control) | 176086 | 97.27 |
| Blank (negative control) | 830 | 0.13 |

### 5. Activation assay of anti-BTN3A1 rabbit-derived monoclonal antibodies on γδ T cells

The amount of IFNγ secreted by Vγ9Vδ2 T cells was quantified using functional assays to evaluate the activation capacity of rabbit-derived B cell supernatants.

Experimental method: Vγ9Vδ2 T cells were co-cultured with A375 target cells and mixed with rabbit-derived B cell supernatants, and the mixtures were incubated for 24 hours in a 5% CO₂ incubator at 37°C. The amount of human IFNγ secreted in the cell supernatants was quantified using an ELISA kit.

The results in Table 4 showed that r17G4 and r20A3 were able to significantly induce Vγ9Vδ2 T cells to produce IFNγ following incubation, indicating that these antibodies are capable of activating Vγ9Vδ2 T cells.

**Table 4. Results of activation assay of anti-BTN3A1 rabbit-derived monoclonal antibodies for Vγ9Vδ2 T cells**

| Sample | IFNγ (pg/mL) |
|---|---|
| Antibody r17G4 | 1657 |
| Antibody r20A3 | 1839 |
| Blank (negative control) | 500 |

### Example 2. Preparation of human-rabbit chimeric antibodies from anti-BTN3A1 rabbit-derived monoclonal antibodies

### 1. Sequencing of anti-BTN3A1 rabbit-derived monoclonal antibodies

B cells corresponding to the above antibodies and in the logarithmic growth phase were harvested. RNA was extracted from the cells using Trizol, which was then reverse-transcribed into cDNA. The cDNA was PCR amplified with rabbit Ig-Primers, and the amplified fragments were subcloned into a vector. Following sequencing, the sequences of the rabbit-derived monoclonal antibodies were finally obtained.

The heavy chain variable region (HCVR) and light chain variable region (LCVR) sequences of rabbit-derived monoclonal antibodies r17G4 and r20A3 are as follows:
>r17G4 HCVR 
QSLEESGGRLVTPGTPLTLTCTASGFSLNDYYITWVRQAPGKGLEWIGVIYG
SSNTVYASWAKGRFTISKTSTMVDLRITSPTTEDTATYFCARGYLASSADIWGP
GTLVTVSL (SEQ ID NO: 1)
>r17G4 LCVR 
QVLTQTPSPVSAAVGGTVTINCQSSQSVYNNNRLAWYQQKPGQPPKLLIYDA
STLASGVPSRFKGSGSGTDFTLTISDLECADAATYYCQTYYSGYIWAFGGGTE
VVVK
(SEQ ID NO: 2)
>r20A3 HCVR 
QSVEESGGRLVTPGTPLTLTCTASGFTINNYYMNWVRQAPGKGLEWIGIIYG
SDNTYYASWAKGRFTISKTSTTVDLIMTSPTTEDTATYFCVRNLDYSSAYFHI
WGPGTLVTVSS (SEQ ID NO: 3)
 >r20A3 LCVR 
QVLTQTPSPVSAAVGGTVSISCQASQSVYNNNRLAWYQQKPGQPPNLLIYEA
SKLASGVPSRFKGSGSGTQFTLIISEVQCEDAATYYCQGYYSGFIYPFGGGTE
VVVK
(SEQ ID NO: 4)
in which the underlined parts are CDR sequences as determined by the Kabat numbering system, as shown in Table 5.

**Table 5. CDR sequences of r17G4 and r20A3 (as determined by the Kabat numbering system)**

| Antibody | Name | Sequence | No. |
|---|---|---|---|
| | HCDR1 | DYYIT | SEQ ID NO: 5 |
| | HCDR2 | VIYGSSNTVYASWAKG | SEQ ID NO: 6 |
| r17G4 | HCDR3 | GYLASSADI | SEQ ID NO: 7 |
| | LCDR1 | QSSQSVYNNNRLA | SEQ ID NO: 8 |
| | LCDR2 | DASTLAS | SEQ ID NO: 9 |
| | LCDR3 | QTYYSGYIWA | SEQ ID NO: 10 |
| r20A3 | HCDR1 | NYYMN | SEQ ID NO: 11 |
| | HCDR2 | IIYGSDNTYYASWAKG | SEQ ID NO: 12 |
| | HCDR3 | NLDYSSAYFHI | SEQ ID NO: 13 |
| | LCDR1 | QASQSVYNNNRLA | SEQ ID NO: 14 |
| | LCDR2 | EASKLAS | SEQ ID NO: 15 |
| | LCDR3 | QGYYSGFIYP | SEQ ID NO: 16 |

The two antibodies have the general CDR structures as follows:
HCDR1 (SEQ ID NO: 17): X₁YYX₂X₃, wherein X₁ is selected from D or N, X₂ is selected from I or M, and X₃ is selected from T or N;
HCDR2 (SEQ ID NO: 18): X₄IYGSX₅NTX₆YASWAKG, wherein X₄ is selected from V or I, X₅ is selected from S or D, and X₆ is selected from V or Y;
HCDR3 (SEQ ID NO: 19): X₇X₈X₉X₁₀SSAX₁₁X₁₂X₁₃X₁₄, wherein X₇ is selected from G or N, X₃ is selected from Y or L, X₉ is selected from L or D, X₁₀ is selected from A or Y, X₁₁ is selected from D or Y, X₁₂ is selected from I or F, X₁₃ is absent or H, and X₁₄ is absent or I;
LCDR1 (SEQ ID NO: 20): QX₁₅SQSVYNNNRLA, wherein X₁₅ is selected from S or A;
LCDR2 (SEQ ID NO: 21): X₁₆ASX₁₇LAS, wherein X₁₆ is selected from D or E, and X₁₇ is selected from T or K; and
LCDR3 (SEQ ID NO: 22): QX₁₈YYSGX₁₉IX₂₀X₂₁, wherein X₁₈ is selected from T or G, X₁₉ is selected from Y or F, X₂₀ is selected from W or Y, and X₂₁ is selected from A or P.

### 2. Construction and preparation of human-rabbit chimeric antibodies

The full-length human-rabbit chimeric antibodies 17G4 and 20A3 were constructed by ligating the obtained heavy chain variable regions and light chain variable regions to the human IgG1 heavy chain constant region (Fc region) harboring the L234F/L235E/P331S mutations (numbered according to the Eu numbering system) and the human Cκ light chain constant region, respectively. The sequences are as follows:
>IgG1 Fc region (L234F/L235E/P331S) 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKRVEPKSC
 
DKTHTCPPCPAPEFEGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPASIEKTISKAKGQPREPQVYTLPPSREEMTKNQVSLTCLVKG
FYPSDIAVEWESNGQPENNYKTTPPVLDSDGSFFLYSKLTVDKSRWQQGN
VFSCSVMHEALHNHYTQKSLSLSPGK (SEQ ID NO: 23)
>IgG1 Fc region (L234F/L235E/P331S, with K446 deleted) 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKRVEPKSC
DKTHTCPPCPAPEFEGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPASIEKTISKAKGQPREPQVYTLPPSREEMTKNQVSLTCLVKG
FYPSDIAVEWESNGQPENNYKTTPPVLDSDGSFFLYSKLTVDKSRWQQGN
VFSCSVMHEALHNHYTQKSLSLSPG (SEQ ID NO: 24)
>17G4 HC 
QSLEESGGRLVTPGTPLTLTCTASGFSLNDYYITWVRQAPGKGLEWIGVIY
GSSNTVYASWAKGRFTISKTSTMVDLRITSPTTEDTATYFCARGYLASSADI
WGPGTLVTVSLASTKGPSVFPLAPSSKSTSGGTAALGCLVKDYFPEPVTVS
WNSGALTSGVHTFPAVLQSSGLYSLSSVVTVPSSSLGTQTYICNVNHKPSN
TKVDKRVEPKSCDKTHTCPPCPAPEFEGGPSVFLFPPKPKDTLMISRTPEVT
CVVVDVSHEDPEVKFNWYVDGVEVHNAKTKPREEQYNSTYRVVSVLTVL
HQDWLNGKEYKCKVSNKALPASIEKTISKAKGQPREPQVYTLPPSREEMT
KNQVSLTCLVKGFYPSDIAVEWESNGQPENNYKTTPPVLDSDGSFFLYSKL
TVDKSRWQQGNVFSCSVMHEALHNHYTQKSLSLSPGK (SEQ ID NO: 25)
>17G4 LC 
QVLTQTPSPVSAAVGGTVTINCQSSQSVYNNNRLAWYQQKPGQPPKLLIY
DASTLASGVPSRFKGSGSGTDFTLTISDLECADAATYYCQTYYSGYIWAFG
GGTEVVVKRTVAAPSVFIFPPSDEQLKSGTASVVCLLNNFYPREAKVQWK
VDNALQSGNSQESVTEQDSKDSTYSLSSTLTLSKADYEKHKVYACEVTHQ
GLSSPVTKSFNRGEC
(SEQ ID NO: 26)
>20A3 HC 
QSVEESGGRLVTPGTPLTLTCTASGFTINNYYMNWVRQAPGKGLEWIGIIY
GSDNTYYASWAKGRFTISKTSTTVDLIMTSPTTEDTATYFCVRNLDYSSAY
FHIWGPGTLVTVSSASTKGPSVFPLAPSSKSTSGGTAALGCLVKDYFPEPVT
 
VSWNSGALTSGVHTFPAVLQSSGLYSLSSVVTVPSSSLGTQTYICNVNHKP
SNTKVDKRVEPKSCDKTHTCPPCPAPEFEGGPSVFLFPPKPKDTLMISRTPE
VTCVVVDVSHEDPEVKFNWYVDGVEVHNAKTKPREEQYNSTYRVVSVLT
VLHQDWLNGKEYKCKVSNKALPASIEKTISKAKGQPREPQVYTLPPSREE
MTKNQVSLTCLVKGFYPSDIAVEWESNGQPENNYKTTPPVLDSDGSFFLYS
KLTVDKSRWQQGNVFSCSVMHEALHNHYTQKSLSLSPGK (SEQ ID NO: 27)
>20A3 LC

QVLTQTPSPVSAAVGGTVSISCQASQSVYNNNRLAWYQQKPGQPPNLLIY
EASKLASGVPSRFKGSGSGTQFTLIISEVQCEDAATYYCQGYYSGFIYPFGG
GTEVVVKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC
(SEQ ID NO: 28)

Also provided here are the sequences of the positive anti-BTN3A1 antibody mAb1 (derived from WO 2020025703, corresponding to SEQ ID NOs: 4 and 6 in this patent), whose heavy chain constant region is the IgG1 Fc region (L234F/L235E/P331 S, with K446 deleted).
>mAb1 HC 
QVQLVQSGAEVKKPGASVKLSCKASGYIFTRYYMYWVKQRPGQGLEWIG
EINPNNGGTKFNEKFKNRATLTVDKSISTAYMELSRLRSDDTAVYYCSRED
DYDGTPFAMDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQ
TYICNVNHKPSNTKVDKRVEPKSCDKTHTCPPCPAPEFEGGPSVFLFPPKPK
DTLMISRTPEVTCVVVDVSHEDPEVKFNWYVDGVEVHNAKTKPREEQYN
STYRVVSVLTVLHQDWLNGKEYKCKVSNKALPASIEKTISKAKGQPREPQ
VYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPG
(SEQ ID NO: 29)
>mAb1 LC

DIQMTQSPSSLSASVGDRVTITCHASQNINVWLSWYQQKPGKAPKLLIYKA
SNLHTGVPSRFTGSGSGTDFTFTISSLQPEDIATYYCQQGQTYPYTFGQGTK
LEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPV
TKSFNRGEC

(SEQ ID NO: 30)

In the above sequences, the underlined part in the full-length heavy chain is the human IgG1 Fc region, and the underlined part in the full-length light chain is the human Cκ region.

The gene sequences encoding the above antibodies were synthesized and subcloned into the pcDNA3.1 expression vector. CHO cells were transfected with the expression vector and the transfection reagent PEI at a ratio of 1 : 2, and then incubated in a CO₂ incubator for 4-5 days. After the supernatants containing the expressed antibodies were collected by centrifugation, the antibodies were purified by conventional methods, and following verification, the antibodies of interest were obtained.

### Example 3. Function and effect validation of anti-BTN3A1 human-rabbit chimeric antibodies

### 1. ELISA binding assay of chimeric antibodies to BTN3A1 recombinant protein

The binding properties of anti-BTN3A1 human-rabbit chimeric antibodies to human BTN3A1 were determined by ELISA.

Experimental method: 96-well ELISA plates were coated with the human BTN3A1 recombinant protein with his tag (h-BTN3A1-his). After various concentrations of anti-BTN3A1 human-rabbit chimeric antibodies were added, the binding activity between the antibody and the antigen was measured by adding a secondary antibody (HRP-conjugated anti-primary antibody Fc antibody) and the HRP substrate TMB. 96-well ELISA plates were coated with the human BTN3A1 recombinant protein with his tag (h-BTN3A1-his) at a concentration of 1 µg/mL, and incubated overnight at 4°C. The coated plates were spun dry and patted dry on absorbent paper, 150 µL of blocking buffer was added per well, and the ELISA plates were incubated for 1 hour at 37°C. The plates were spun dry and patted dry on absorbent paper. The anti-BTN3A1 antibodies to be tested were added to each well, and the plates were incubated for 1 hour at 37°C. The supernatants were discarded, and the plates were patted dry. 100 µL of HRP-labeled anti-human IgG secondary antibody (diluted 1 : 5000 in dilution buffer) was added per well, and the plates were incubated for 1 hour at 37°C. The ELISA plates were washed with a plate washer using PBS, with a program of 5 washing cycles and a 30-second soak per cycle. Subsequently, 100 µL of substrate solution (TMB) was added per well, and the mixtures were reacted for 5 minutes to allow color development. The reactions were finally terminated by adding 100 µL of 1 mol/L sulfuric acid per well. The OD value was measured using a microplate reader (Tecan-infinite F50) at a wavelength of 450 nm.

The results are shown in Table 6 and Figure 1. IgG1 is an isotype control antibody, as in other examples of the present invention. The results showed that 17G4 and 20A3 exhibited significant binding to the human BTN3A1 recombinant protein, and their binding activity was comparable to that of mAb1.

**Table 6. Results of ELISA binding assay of anti-BTN3A1 human-rabbit chimeric antibodies to human BTN3A1 recombinant protein**

| Antibody | EC₅₀ for binding to human BTN3A1 (nM) |
|---|---|
| 17G4 | 0.02283 |
| 20A3 | 0.01963 |
| mAb1 | 0.02181 |
| IgG1 | No-binding |

### 2. FACS binding assay of chimeric antibodies to BTN3A1-overexpressing cells

The binding properties of anti-BTN3A1 human-rabbit chimeric antibodies were determined by flow cytometry (FACS).

Experimental method: A cell line transiently overexpressing human BTN3A1 with endogenous BTN3A protein knocked out was constructed. After the addition of antibodies, the binding properties of the antibodies to the antigen were determined by adding a secondary antibody.

The process for constructing the cell line was as follows: HEK293 cells were infected with lentiviruses carrying short hairpin RNA sequences with human BTN3A (including BTN3A1, BTN3A2, and BTN3A3) knocked out, and a stably-transfected monoclonal cell line overexpressing human BTN3A1 was obtained via antibiotic selection and infinite dilution. Subsequently, expression plasmids carrying the human BTN3A1 gene sequence were transfected into the cells with endogenous BTN3A knocked out, and a stably-transfected monoclonal cell line overexpressing human BTN3A1 was obtained via antibiotic selection and infinite dilution.

2 × 10⁵ overexpressing cells were seeded per well in 96-well plates. The plates were centrifuged for 5 minutes at 300 g, and the supernatants were discarded. 100 µL of antibodies to be tested were added per well, and the plates were incubated for 1 hour at 4°C. The supernatants were discarded by centrifugation, and the plates were washed three times with 200 µL of washing buffer (PBS + 2% FBS). Then, 100 µL of Alexa Fluor 488-labeled anti-human IgG secondary antibody (Invitrogen, A-11013; diluted 1 : 500) was added, and the plates were incubated for 1 hour at 4°C. The supernatants were discarded by centrifugation, and the plates were washed three times with 200 µL of washing buffer (PBS + 2% FBS). Finally, the cells were resuspended in 100 µL of PBS and analyzed by flow cytometry.

The results are shown in Table 7 and Figure 2. The results showed that 17G4 and 20A3 exhibited significant binding to human BTN3A1-overexpressing cells, and their binding intensities were both slightly stronger than that of mAb1.

**Table 7. Results of binding assay of anti-BTN3A1 human-rabbit chimeric antibodies to human BTN3A1-overexpressing cells**

| Antibody | FACS EC₅₀ for human BTN3A1 (nM) |
|---|---|
| 17G4 | 15.45 |
| 20A3 | 8.199 |
| mAb1 | 21.19 |
| IgG1 | No-binding |

### 3. Affinity determination of chimeric antibodies for BTN3A1 recombinant protein

Experimental method: The determination was performed using a Biacore 8K instrument (GE Healthcare). A Protein A sensor chip was selected, and HBS-EP+ buffer (10 mM HEPES, 150 mM NaCl, 3 mM EDTA, and 0.05% surfactant P20) was used as the mobile phase. Each antibody to be tested was prepared as the ligand with HBS-EP+ buffer, respectively, and captured by the Protein A protein on the chip channel. The human BTN3A1 antigen protein (Kactus Biosystems, BTN-HM1A1) was used as the analyte and prepared with HBS-EP+ buffer. The analyte was subjected to 2-fold gradient dilution starting from 100 nM, resulting in a total of seven concentration points. It was injected through the sample channel and reference channel at a flow rate of 30 µL/min, with an association time of 90 s and a dissociation time of 500 s. The regeneration buffer (10 mM Glycine, pH 1.5; GE Healthcare, 29238268-AA) was run for 30 s at a flow rate of 10 µl/min. The data was processed with the Biacore 8K Evaluation analysis software. The signal values of the sample channel (Fc 2) were subtracted from those of the corresponding reference channel (Fc 1), yielding the corrected signal curves. The affinity kinetic curves were fitted to the 1 : 1 Langmuir binding model, and the association rate constant (Ka), dissociation rate constant (Kd), and dissociation constant (i.e., affinity KD value) were calculated.

The affinity of anti-BTN3A1 antibodies for human BTN3A1 is shown in Table 8. The results showed that 17G4 and 20A3 bound to the human BTN3A1 protein with high affinity, and their affinity was stronger than that of mAb1 for human BTN3A1.

**Table 8. Affinity of anti-BTN3A1 human-rabbit chimeric antibodies for human BTN3A1**

| Antibody | Kₒₙ (1/Ms) | K_{off} (1/s) | K_{D} (M) |
|---|---|---|---|
| 17G4 | 8.00E+05 | 1.82E-04 | 2.28E-10 |
| 20A3 | 9.85E+05 | 1.72E-04 | 1.74E-10 |
| mAb1 | 1.08E+05 | 1.18E-04 | 1.09E-09 |

### 4. Activation assay of chimeric antibodies on γδ T cells

The *in vitro* activation function of anti-BTN3A1 human-rabbit chimeric antibodies on γδ T cells was evaluated by quantifying the amount of IFNγ secreted by γδ T cells.

Experimental method: Vγ9Vδ2 T cells were co-cultured with A375 target cells, followed by the addition of gradient concentrations of anti-BTN3A1 human-rabbit chimeric antibodies, and the mixtures were incubated in a 5% CO₂ incubator at 37°C. The amount of human IFNγ secreted in the cell supernatants was quantified using an ELISA kit.

The results are shown in Table 9 and Figure 3, and 17G4 and 20A3 were found to significantly activate Vγ9Vδ2 T cells.

**Table 9. Results of activation assay of anti-BTN3A1 human-rabbit chimeric antibodies on γδ T cells**

| Antibody | EC₅₀ (nM) | Max IFN-γ (pg/mL) |
|---|---|---|
| 17G4 | 1.389 | 1540 |
| 20A3 | 0.8575 | 949.2 |
| mAb1 | 1.140 | 1061 |
| IgG1 | Inactive | Inactive |

### Example 4. Humanization engineering of anti-BTN3A1 rabbit-derived antibodies

This example compared the heavy and light chain variable region sequences with antibody germline databases to obtain human germline templates with high homology, based on the typical VH/VL structures of the obtained rabbit-derived monoclonal antibodies 17G4 and 20A3. The human germline heavy chain framework regions were derived from the human heavy chain, and the human germline light chain framework regions were derived from the human κ light chain. The human germline heavy chain template IGHV3-66 and the human germline light chain template IGkV1-27 were preferred for the antibodies 17G4 and 20A3 of the present invention. The CDR regions of the rabbit-derived monoclonal antibodies were grafted onto the selected humanization templates, replacing the endogenous CDR regions of the humanization templates, thereby constructing the variable regions. Subsequently, based on the three-dimensional structures of the rabbit-derived monoclonal antibodies, back mutations were performed on buried residues, residues with direct interactions with the CDR regions, and residues that exert a significant impact on the conformations of VH and VL, and the chemically unstable amino acid residues in the CDR regions were optimized, thereby generating a series of humanized antibodies.

The heavy chain variable regions of each of the 17G4's humanized antibodies are as follows:
>17G4 H1CVR 
EVQLVESGGGLVQPGGSLRLSCAASGFSLNDYYITWVRQAPGKGLEWVSV
IYGSSNTVYASWAKGRFTISKDNSKNTVYLQMNSLRAEDTAVYYCARGY
LASSADIWGQGTLVTVSS
(SEQ ID NO: 31)
>17G4 H2CVR 
EVQLVESGGGLVQPGGSLRLSCAASGFSLNDYYITWVRQAPGKGLEWVSV
IYGSSNTVYASWAKGRFTISKDNSKTTVYLQMNSLRAEDTAVYFCARGYL
ASSADIWGQGTLVTVSS
(SEQ ID NO: 32)
>17G4 H3CVR 
EQQLVESGGGLVQPGGSLRLSCAASGFSLNDYYITWVRQAPGKGLEWVSV
IYGSSNTVYASWAKGRFTISKDNSKTTVYLQMNSLRAEDTAVYFCARGYL
ASSADIWGQGTLVTVSS
(SEQ ID NO: 33)
>17G4 H4CVR 
EQQLVESGGGLVQPGGSLRLSCAASGFSLNDYYITWVRQAPGKGLEWIGV
IYGSSNTVYASWAKGRFTISKDNSKTTVYLQMNSLRAEDTAVYFCARGYL
ASSADIWGQGTLVTVSS
(SEQ ID NO: 34)
>17G4 H5CVR

EQQLVESGGGLVQPGGSLRLSCAASGFSLNDYYITWVRQAPGKGLEWIGV
IYGSSNTVYASWAKGRFTISKTSTMVYLQMNSLRAEDTAVYFCARGYLAS
SADIWGQGTLVTVSS
(SEQ ID NO: 35)

The light chain variable regions of each of the 17G4's humanized antibodies are as follows:
>17G4 L1CVR 
DIQLTQSPSSLSASVGDRVTITCQSSQSVYNNNRLAWYQQKPGKVPKLLIY
DASTLASGVPSRFSGSGSGTDFTLTISSLQPEDVATYYCQTYYSGYIWAFG
GGTKVEIK
(SEQ ID NO: 36)
>17G4 L2CVR 
DIQLTQSPSSLSASVGDRVTITCQSSQSVYNNNRLAWYQQKPGKPPKLLIY
DASTLASGVPSRFSGSGSGTDFTLTISSLQPEDVATYYCQTYYSGYIWAFG
GGTKVEIK
(SEQ ID NO: 37)
>17G4 L3CVR

DQQLTQSPSSLSASVGDRVTITCQSSQSVYNNNRLAWYQQKPGKPPKLLIY
DASTLASGVPSRFSGSGSGTDFTLTISSLQPEDVATYYCQTYYSGYIWAFG
GGTKVEIK (SEQ ID NO: 38)

In the above sequences, the underlined parts are CDRs, as in SEQ ID NOs: 5-10.

The heavy chain variable regions of each of the 20A3's humanized antibodies are as follows:
>20A3 H1CVR 
EVQLVESGGGLVQPGGSLRLSCAASGFTINNYYMNWVRQAPGKGLEWVS
IIYGSDNTYYASWAKGRFTISRDNSKNTLYLQMNSLRAEDTAVYYCVRNL
DYSSAYFHIWGQGTLVTVSS
(SEQ ID NO: 39)
>20A3 H2CVR 
EVQLVESGGGLVQPGGSLRLSCAASGFTINNYYMNWVRQAPGKGLEWVS
IIYGSDNTYYASWAKGRFTISKDNSKNTVYLQMNSLRAEDTAVYYCVRNL
DYSSAYFHIWGQGTLVTVSS
(SEQ ID NO: 40)
>20A3 H3CVR 
EVQVVESGGGLVQPGGSLRLSCAASGFTINNYYMNWVRQAPGKGLEWVS
IIYGSDNTYYASWAKGRFTISKDNSKTTVYLQMNSLRAEDTAVYFCVRNL
DYSSAYFHIWGQGTLVTVSS
(SEQ ID NO: 41)
>20A3 H4CVR 
EQQVVESGGGLVQPGGSLRLSCAASGFTINNYYMNWVRQAPGKGLEWVS
IIYGSDNTYYASWAKGRFTISKDNSKTTVYLQMNSLRAEDTAVYFCVRNL
DYSSAYFHIWGQGTLVTVSS
(SEQ ID NO: 42)
>20A3 H5CVR 
EQQVVESGGGLVQPGGSLRLSCAASGFTINNYYMNWVRQAPGKGLEWIGI
IYGSDNTYYASWAKGRFTISKDNSKTTVYLQMNSLRAEDTAVYFCVRNLD
YSSAYFHIWGQGTLVTVSS
(SEQ ID NO: 43)
>20A3 H6CVR

EQQVVESGGGLVQPGGSLRLSCAASGFTINNYYMNWVRQAPGKGLEWIGI
IYGSDNTYYASWAKGRFTISKTSTTVYLQMNSLRAEDTAVYFCVRNLDYS
SAYFHIWGQGTLVTVSS
(SEQ ID NO: 44)

The light chain variable regions of each of the 20A3's humanized antibodies are as follows:
>20A3 L1CVR 
DIQLTQSPSSLSASVGDRVTITCQASQSVYNNNRLAWYQQKPGKVPKLLIY
EASKLASGVPSRFSGSGSGTDFTLTISSLQPEDVATYYCQGYYSGFIYPFGG
GTKVEIK
(SEQ ID NO: 45)
>20A3 L2CVR 
DIQLTQSPSSLSASVGDRVTITCQASQSVYNNNRLAWYQQKPGKPPKLLIY
EASKLASGVPSRFSGSGSGTDFTLTISSLQPEDVATYYCQGYYSGFIYPFGG
GTKVEIK
(SEQ ID NO: 46)
>20A3 L3CVR

DQQLTQSPSSLSASVGDRVTITCQASQSVYNNNRLAWYQQKPGKPPKLLIY
EASKLASGVPSRFSGSGSGTDFTLTISSLQPEDVATYYCQGYYSGFIYPFGG
GTKVEIK
(SEQ ID NO: 47)

In the above sequences, the underlined parts are CDRs, as in SEQ ID NOs: 11-16.

The full-length sequences were obtained by ligating each of the above heavy chain variable regions and each of the above light chain variable regions to the human IgG1 heavy chain constant region harboring the L234F/L235E/P331S mutations and the human Cκ light chain constant region, respectively.

The full-length heavy chain sequences of each of the 17G4's humanized antibodies are as follows:
>17G4 H1C 
EVQLVESGGGLVQPGGSLRLSCAASGFSLNDYYITWVRQAPGKGLEWVSV
IYGSSNTVYASWAKGRFTISKDNSKNTVYLQMNSLRAEDTAVYYCARGY
LASSADIWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNV
 
NHKPSNTKVDKRVEPKSCDKTHTCPPCPAPEFEGGPSVFLFPPKPKDTLMIS
RTPEVTCVVVDVSHEDPEVKFNWYVDGVEVHNAKTKPREEQYNSTYRVV
SVLTVLHQDWLNGKEYKCKVSNKALPASIEKTISKAKGQPREPQVYTLPPS
REEMTKNQVSLTCLVKGFYPSDIAVEWESNGQPENNYKTTPPVLDSDGSFF
LYSKLTVDKSRWQQGNVFSCSVMHEALHNHYTQKSLSLSPGK
(SEQ ID NO: 48)
>17G4 H2C 
EVQLVESGGGLVQPGGSLRLSCAASGFSLNDYYITWVRQAPGKGLEWVSV
IYGSSNTVYASWAKGRFTISKDNSKTTVYLQMNSLRAEDTAVYFCARGYL
ASSADIWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNV
NHKPSNTKVDKRVEPKSCDKTHTCPPCPAPEFEGGPSVFLFPPKPKDTLMIS
RTPEVTCVVVDVSHEDPEVKFNWYVDGVEVHNAKTKPREEQYNSTYRVV
SVLTVLHQDWLNGKEYKCKVSNKALPASIEKTISKAKGQPREPQVYTLPPS
REEMTKNQVSLTCLVKGFYPSDIAVEWESNGQPENNYKTTPPVLDSDGSFF
LYSKLTVDKSRWQQGNVFSCSVMHEALHNHYTQKSLSLSPGK
(SEQ ID NO: 49)
>17G4 H3C 
EQQLVESGGGLVQPGGSLRLSCAASGFSLNDYYITWVRQAPGKGLEWVSV
IYGSSNTVYASWAKGRFTISKDNSKTTVYLQMNSLRAEDTAVYFCARGYL
ASSADIWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNV
NHKPSNTKVDKRVEPKSCDKTHTCPPCPAPEFEGGPSVFLFPPKPKDTLMIS
RTPEVTCVVVDVSHEDPEVKFNWYVDGVEVHNAKTKPREEQYNSTYRVV
SVLTVLHQDWLNGKEYKCKVSNKALPASIEKTISKAKGQPREPQVYTLPPS
REEMTKNQVSLTCLVKGFYPSDIAVEWESNGQPENNYKTTPPVLDSDGSFF
LYSKLTVDKSRWQQGNVFSCSVMHEALHNHYTQKSLSLSPGK
(SEQ ID NO: 50)
>17G4 H4C 
EQQLVESGGGLVQPGGSLRLSCAASGFSLNDYYITWVRQAPGKGLEWIGV
IYGSSNTVYASWAKGRFTISKDNSKTTVYLQMNSLRAEDTAVYFCARGYL
ASSADIWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNV
NHKPSNTKVDKRVEPKSCDKTHTCPPCPAPEFEGGPSVFLFPPKPKDTLMIS
 
RTPEVTCVVVDVSHEDPEVKFNWYVDGVEVHNAKTKPREEQYNSTYRVV
SVLTVLHQDWLNGKEYKCKVSNKALPASIEKTISKAKGQPREPQVYTLPPS
REEMTKNQVSLTCLVKGFYPSDIAVEWESNGQPENNYKTTPPVLDSDGSFF
LYSKLTVDKSRWQQGNVFSCSVMHEALHNHYTQKSLSLSPGK
(SEQ ID NO: 51)
>17G4 H5C

EQQLVESGGGLVQPGGSLRLSCAASGFSLNDYYITWVRQAPGKGLEWIGV
IYGSSNTVYASWAKGRFTISKTSTMVYLQMNSLRAEDTAVYFCARGYLAS
SADIWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNH
KPSNTKVDKRVEPKSCDKTHTCPPCPAPEFEGGPSVFLFPPKPKDTLMISRT
PEVTCVVVDVSHEDPEVKFNWYVDGVEVHNAKTKPREEQYNSTYRVVSV
LTVLHQDWLNGKEYKCKVSNKALPASIEKTISKAKGQPREPQVYTLPPSRE
EMTKNQVSLTCLVKGFYPSDIAVEWESNGQPENNYKTTPPVLDSDGSFFL
YSKLTVDKSRWQQGNVFSCSVMHEALHNHYTQKSLSLSPGK
(SEQ ID NO: 52)

The full-length light chain sequences of each of the 17G4's humanized antibodies are as follows:
>17G4 L1C 
DIQLTQSPSSLSASVGDRVTITCQSSQSVYNNNRLAWYQQKPGKVPKLLIY
DASTLASGVPSRFSGSGSGTDFTLTISSLQPEDVATYYCQTYYSGYIWAFG
GGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC
(SEQ ID NO: 53)
>17G4 L2C 
DIQLTQSPSSLSASVGDRVTITCQSSQSVYNNNRLAWYQQKPGKPPKLLIY
DASTLASGVPSRFSGSGSGTDFTLTISSLQPEDVATYYCQTYYSGYIWAFG
GGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC
(SEQ ID NO: 54)
>17G4 L3C

DQQLTQSPSSLSASVGDRVTITCQSSQSVYNNNRLAWYQQKPGKPPKLLIY
DASTLASGVPSRFSGSGSGTDFTLTISSLQPEDVATYYCQTYYSGYIWAFG
GGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQG
LSSPVTKSFNRGEC
(SEQ ID NO: 55)

The full-length heavy chain sequences of each of the 20A3's humanized antibodies are as follows:
>20A3 H1C 
EVQLVESGGGLVQPGGSLRLSCAASGFTINNYYMNWVRQAPGKGLEWVS
IIYGSDNTYYASWAKGRFTISRDNSKNTLYLQMNSLRAEDTAVYYCVRNL
DYSSAYFHIWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYIC
NVNHKPSNTKVDKRVEPKSCDKTHTCPPCPAPEFEGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPASIEKTISKAKGQPREPQVYT
LPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
(SEQ ID NO: 56)
>20A3 H2C 
EVQLVESGGGLVQPGGSLRLSCAASGFTINNYYMNWVRQAPGKGLEWVS
IIYGSDNTYYASWAKGRFTISKDNSKNTVYLQMNSLRAEDTAVYYCVRNL
DYSSAYFHIWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYIC
NVNHKPSNTKVDKRVEPKSCDKTHTCPPCPAPEFEGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPASIEKTISKAKGQPREPQVYT
LPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
(SEQ ID NO: 57)
>20A3 H3C 
EVQVVESGGGLVQPGGSLRLSCAASGFTINNYYMNWVRQAPGKGLEWVS
IIYGSDNTYYASWAKGRFTISKDNSKTTVYLQMNSLRAEDTAVYFCVRNL
DYSSAYFHIWGQGTLVTVSSASTKGPSVFPLAPSSKSTSGGTAALGCLVKD
 
YFPEPVTVSWNSGALTSGVHTFPAVLQSSGLYSLSSVVTVPSSSLGTQTYIC
NVNHKPSNTKVDKRVEPKSCDKTHTCPPCPAPEFEGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPASIEKTISKAKGQPREPQVYT
LPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
(SEQ ID NO: 58)
>20A3 H4C 
EQQVVESGGGLVQPGGSLRLSCAASGFTINNYYMNWVRQAPGKGLEWVS
IIYGSDNTYYASWAKGRFTISKDNSKTTVYLQMNSLRAEDTAVYFCVRNL
DYSSAYFHIWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYIC
NVNHKPSNTKVDKRVEPKSCDKTHTCPPCPAPEFEGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPASIEKTISKAKGQPREPQVYT
LPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
(SEQ ID NO: 59)
>20A3 H5C 
EQQVVESGGGLVQPGGSLRLSCAASGFTINNYYMNWVRQAPGKGLEWIGI
IYGSDNTYYASWAKGRFTISKDNSKTTVYLQMNSLRAEDTAVYFCVRNLD
YSSAYFHIWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICN
VNHKPSNTKVDKRVEPKSCDKTHTCPPCPAPEFEGGPSVFLFPPKPKDTLM
ISRTPEVTCVVVDVSHEDPEVKFNWYVDGVEVHNAKTKPREEQYNSTYR
VVSVLTVLHQDWLNGKEYKCKVSNKALPASIEKTISKAKGQPREPQVYTL
PPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK
(SEQ ID NO: 60)
>20A3 H6C

EQQVVESGGGLVQPGGSLRLSCAASGFTINNYYMNWVRQAPGKGLEWIGI
IYGSDNTYYASWAKGRFTISKTSTTVYLQMNSLRAEDTAVYFCVRNLDYS
SAYFHIWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNV

NHKPSNTKVDKRVEPKSCDKTHTCPPCPAPEFEGGPSVFLFPPKPKDTLMIS
RTPEVTCVVVDVSHEDPEVKFNWYVDGVEVHNAKTKPREEQYNSTYRVV
SVLTVLHQDWLNGKEYKCKVSNKALPASIEKTISKAKGQPREPQVYTLPPS
REEMTKNQVSLTCLVKGFYPSDIAVEWESNGQPENNYKTTPPVLDSDGSFF
LYSKLTVDKSRWQQGNVFSCSVMHEALHNHYTQKSLSLSPGK
(SEQ ID NO: 61)

The full-length light chain sequences of each of the 20A3's humanized antibodies are as follows:
>20A3 L1C 
DIQLTQSPSSLSASVGDRVTITCQASQSVYNNNRLAWYQQKPGKVPKLLIY
EASKLASGVPSRFSGSGSGTDFTLTISSLQPEDVATYYCQGYYSGFIYPFGG
GTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGL
SSPVTKSFNRGEC
(SEQ ID NO: 62)
>20A3 L2C 
DIQLTQSPSSLSASVGDRVTITCQASQSVYNNNRLAWYQQKPGKPPKLLIY
EASKLASGVPSRFSGSGSGTDFTLTISSLQPEDVATYYCQGYYSGFIYPFGG
GTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGL
SSPVTKSFNRGEC
(SEQ ID NO: 63)
>20A3 L3C

DQQLTQSPSSLSASVGDRVTITCQASQSVYNNNRLAWYQQKPGKPPKLLIY
EASKLASGVPSRFSGSGSGTDFTLTISSLQPEDVATYYCQGYYSGFIYPFGG
GTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGL
SSPVTKSFNRGEC
(SEQ ID NO: 64)

The above full-length humanized heavy chain sequences and full-length humanized light chain sequences were paired, respectively, thereby obtaining the following humanized molecules:

**Table 10. Anti-BTN3A1 humanized antibodies**

| Antibody | Heavy chain | Light chain |
|---|---|---|
| 17G4 (a human-rabbit chimeric antibody, parent) | 17G4 HC (SEQ ID NO: 25) | 17G4 LC (SEQ ID NO: 26) |
| 17G4-H1L1 | 17G4 H1C (SEQ ID NO: 48) | 17G4 L1C (SEQ ID NO: 53) |
| 17G4-H2L1 | 17G4 H2C (SEQ ID NO: 49) | |
| 17G4-H3L1 | 17G4 H3C (SEQ ID NO: 50) | |
| 17G4-H1L2 | 17G4 H1C (SEQ ID NO: 48) | 17G4 L2C (SEQ ID NO: 54) |
| 17G4-H2L2 | 17G4 H2C (SEQ ID NO: 49) | |
| 17G4-H3L2 | 17G4 H3C (SEQ ID NO: 50) | |
| 17G4-H1L3 | 17G4 H1C (SEQ ID NO: 48) | 17G4 L3C (SEQ ID NO: 55) |
| 17G4-H2L3 | 17G4 H2C (SEQ ID NO: 49) | |
| 17G4-H3L3 | 17G4 H3C (SEQ ID NO: 50) | |
| 17G4-H4L3 | 17G4 H4C (SEQ ID NO: 51) | |
| 17G4-H5L3 | 17G4 H5C (SEQ ID NO: 52) | |
| 20A3 (a human-rabbit chimeric antibody, parent) | 20A3 HC (SEQ ID NO: 27) | 20A3 LC (SEQ ID NO: 28) |
| 20A3-H1L1 | 20A3 H1C (SEQ ID NO: 56) | 20A3 L1C (SEQ ID NO: 62) |
| 20A3-H2L1 | 20A3 H2C (SEQ ID NO: 57) | |
| 20A3-H3L1 | 20A3 H3C (SEQ ID NO: 58) | |
| 20A3-H4L1 | 20A3 H4C (SEQ ID NO: 59) | |
| 20A3-H1L2 | 20A3 H1C (SEQ ID NO: 56) | 20A3 L2C (SEQ ID NO: 63) |
| 20A3-H2L2 | 20A3 H2C (SEQ ID NO: 57) | |
| 20A3-H3L2 | 20A3 H3C (SEQ ID NO: 58) | |
| 20A3-H4L2 | 20A3 H4C (SEQ ID NO: 59) | |
| 20A3-H1L3 | 20A3 H1C (SEQ ID NO: 56) | 20A3 L3C (SEQ ID NO: 64) |
| 20A3-H2L3 | 20A3 H2C (SEQ ID NO: 57) | |
| 20A3-H3L3 | 20A3 H3C (SEQ ID NO: 58) | |
| 20A3-H4L3 | 20A3 H4C (SEQ ID NO: 59) | |
| 20A3-H5L3 | 20A3 H5C (SEQ ID NO: 60) | |
| 20A3-H6L3 | 20A3 H6C (SEQ ID NO: 61) | |

### Example 5. Function and effect validation of anti-BTN3A1 humanized antibodies

### 1. Activation assay of antibodies on γδ T cells

The above humanized molecules of 17G4 and 20A3 were expressed and purified, and the activation capacity of the humanized antibodies on γδ T cells was evaluated in accordance with the method described in Part 4 of Example 3.

The results are shown in Table 11 and Figure 5. The results showed that the activation activities of all humanized molecules of 20A3 did not show any significant reduction compared with the parental 20A3.

**Table 11. Results of activation assay of 20A3's humanized antibodies on γδ T cells**

| Antibody | EC₅₀ (nM) |
|---|---|
| 20A3 | 0.9583 |
| 20A3-H1L1 | 2.641 |
| 20A3-H2L1 | 1.354 |
| 20A3-H3L1 | 2.602 |
| 20A3-H4L1 | 2.514 |
| 20A3-H1L2 | 2.302 |
| 20A3-H2L2 | 1.102 |
| 20A3-H3L2 | 1.920 |
| 20A3-H4L2 | 1.435 |
| 20A3-H1L3 | 1.587 |
| 20A3-H2L3 | 1.648 |
| 20A3-H3L3 | 1.484 |
| 20A3-H4L3 | 1.502 |
| 20A3-H5L3 | 1.185 |
| 20A3-H6L3 | 0.816 |
| mAb1 | 2.865 |

### 2. Binding assay of antibodies to BTN3A1-overexpressing cells

The binding ability of the anti-BTN3A1 humanized antibodies to human BTN3A1-overexpressing cells was determined in accordance with the method described in Part 2 of Example 3.

The results are shown in Table 12, Figure 6A, and Figure 6B, and the binding ability of the humanized molecules of 17G4 and 20A3 to the cells was found to be comparable to that of their respective chimeric antibodies and superior to that of the control antibody.

**Table 12. Results of binding assay of 17G4's and 20A3's humanized antibodies to human BTN3A1-overexpressing cells**

| Antibody | FACS EC₅₀ for human BTN3A1 (nM) | Max FACS mean fluorescence intensity (MFI) for human BTN3A1 |
|---|---|---|
| 17G4 | 17.37 | 97974 |
| 17G4-H5L3 | 19.83 | 89676 |
| 20A3 | 11.65 | 95819 |
| 20A3-H2L2 | 10.20 | 91243 |
| mAb1 | 36.82 | 99709 |
| IgG1 | No-binding | No-binding |

### 3. Affinity determination of antibodies for BTN3A1 recombinant protein

The affinity of 20A3-H2L2 for the human BTN3A1 recombinant protein was determined in accordance with the method described in Part 3 of Example 3.

The results are shown in Table 13. The results showed that 20A3-H2L2 exhibited high binding affinity for the human BTN3A1 recombinant protein, and the affinity of 20A3-H2L2 for human BTN3A1 was stronger than that of mAb1.

**Table 13. Affinity of 20A3-H2L2 for human BTN3A1 recombinant protein**

| Antibody | Human BTN3A1 | | |
|---|---|---|---|
| | Kₒₙ (1/Ms) | K_{off} (1/s) | K_{D} (M) |
| 20A3-H2L2 | 9.85E+05 | 1.72E-04 | 1.74E-10 |
| mAb1 | 1.08E+05 | 1.18E-04 | 1.09E-09 |

### 4. Binding assay of antibodies to BTN3A-overexpressing cells

The binding ability of 20A3-H2L2 to other proteins of the BTN3A family was determined by flow cytometry (FACS). HEK293 cell lines overexpressing human BTN3A2 and human BTN3A3 with endogenous BTN3A protein knocked out were constructed in accordance with the method described in Part 2 of Example 3. Subsequently, the binding properties of 20A3-H2L2 to the HEK293 cells overexpressing human BTN3A1, human BTN3A2, and human BTN3A3 with endogenous BTN3A protein knocked out were determined, respectively.

The results are shown in Table 14, Figure 7A, Figure 7B, and Figure 7C. The results showed that both 20A3-H2L2 and mAb1 were able to bind to human BTN3A1, human BTN3A2, and human BTN3A3, and the binding ability of 20A3-H2L2 was stronger than that of mAb1 in all cases.

**Table 14. Results of binding assay of 20A3-H2L2 to cells overexpressing BTN3A family proteins**

| Test item | Antibody | | |
|---|---|---|---|
| | 20A3-H2L2 | mAb1 | IgG1 |
| FACS EC₅₀ for human BTN3A1 (nM) | 2.979 | 12.310 | No-binding |
| Max FACS mean fluorescence intensity (MFI) for human BTN3A1 | 79089 | 78017 | No-binding |
| FACS EC₅₀ for human BTN3A2 (nM) | 6.760 | 18.25 | No-binding |
| Max FACS mean fluorescence intensity (MFI) for human BTN3A2 | 122205 | 115012 | No-binding |
| FACS EC₅₀ for human BTN3A3 (nM) | 5.122 | 12.51 | No-binding |
| Max FACS mean fluorescence intensity (MFI) for human BTN3A3 | 94368 | 89496 | No-binding |

### 5. Activation assay of antibodies on γδ T cells

The *in vitro* activation function of 20A3-H2L2 on γδ T cells was evaluated by quantifying the amount of IFNγ secreted by γδ T cells. Vγ9Vδ2 T cells were co-cultured with SKOV-3 cells highly expressing BTN3A protein *in vitro* and added with gradient concentrations of the antibody to be tested, and the mixtures were incubated in a 5% CO₂ incubator for 24 hours at 37°C. The amount of human IFNγ secreted in the cell supernatants was quantified using an ELISA kit.

The results are shown in Table 15 and Figure 8. The results showed that the activation capacity of 20A3-H2L2 on Vγ9Vδ2 T cells was stronger than that of mAb1, with a 2.6-fold decrease in EC₅₀. Compared with the experimental results in Part 1 of Example 5 (which used A375 cells with low BTN3A expression), 20A3-H2L2 exhibited stronger activation activity than mAb1 in the present example, indicating that higher BTN3A expression on tumor cells help to further enhance the activation activity of 20A3-H2L2 on Vγ9Vδ2 T cells.

**Table 15. Results of activation assay of 20A3-H2L2 on γδ T cells**

| Antibody | EC₅₀ (nM) | Maximum (pg/mL) |
|---|---|---|
| 20A3-H2L2 | 0.2841 | 2473 |
| mAb1 | 0.7366 | 1762 |
| IgG1 | Inactive | Inactive |

### 6. Assay for γδ T cell-mediated killing of SKOV-3 cells induced by antibodies

The ability of 20A3-H2L2 to mediate killing of target cells by γδ T cells was evaluated by measuring the killing of γδ T cells against the tumor target cells. Vγ9Vδ2 T cells and SKOV-3 cells (luc-labeled) were co-seeded into 96-well plates, followed by the addition of gradient concentrations of 20A3-H2L2, mAb1, or the isotype control (IgG1). After 24 hours of co-culture, the cells were lysed with the Bright-GloTM reagent (Promega) for 5 minutes, and the luminescent activity was measured using an Envision multimode microplate reader (Perkinelmer).

The results are shown in Table 16 and Figure 9. The results showed that the killing ability of Vγ9Vδ2 T cells against SKOV-3 cells induced by 20A3-H2L2 was stronger than that induced by mAb1, and the EC₅₀ of 20A3-H2L2 was about 1/3 of the EC₅₀ of the control antibody.

**Table 16. Results of assay for γδ T cell-mediated killing of SKOV-3 cells induced by 20A3-H2L2**

| Antibody | EC₅₀ (nM) | Max killing rate (%) |
|---|---|---|
| 20A3-H2L2 | 0.1109 | 98.15 |
| mAb1 | 0.3487 | 97.67 |
| IgG1 | Inactive | Inactive |

## Claims

1. A BTN3A-binding protein, comprising a domain that specifically binds to BTN3A and comprises a heavy chain variable region and a light chain variable region, wherein:
the heavy chain variable region comprises HCDR1, HCDR2, and HCDR3 in a heavy chain variable region of the amino acid sequence as set forth in any one of SEQ ID NOs: 1, 3, 31-35, and 39-44, and
the light chain variable region comprises LCDR1, LCDR2, and LCDR3 in a light chain variable region of the amino acid sequence as set forth in any one of SEQ ID NOs: 2, 4, 36-38, and 45-47,
wherein the CDRs are defined according to the Kabat, IMGT, Chothia, AbM, or Contact numbering system;
preferably,
1) the heavy chain variable region comprises HCDR1, HCDR2, and HCDR3 in a heavy chain variable region of the amino acid sequence as set forth in any one of SEQ ID NOs: 3 and 39-44, and
the light chain variable region comprises LCDR1, LCDR2, and LCDR3 in a light chain variable region of the amino acid sequence as set forth in any one of SEQ ID NOs: 4 and 45-47; or
2) the heavy chain variable region comprises HCDR1, HCDR2, and HCDR3 in a heavy chain variable region of the amino acid sequence as set forth in any one of SEQ ID NOs: 1 and 31-35, and
the light chain variable region comprises LCDR1, LCDR2, and LCDR3 in a light chain variable region of the amino acid sequence as set forth in any one of SEQ ID NOs: 2 and 36-38; and
more preferably,
1) the heavy chain variable region comprises HCDR1, HCDR2, and HCDR3 of the amino acid sequences as set forth in SEQ ID NOs: 11-13, respectively, and the light chain variable region comprises LCDR1, LCDR2, and LCDR3 of the amino acid sequences as set forth in SEQ ID NOs: 14-16, respectively; or
2) the heavy chain variable region comprises HCDR1, HCDR2, and HCDR3 of the amino acid sequences as set forth in SEQ ID NOs: 5-7, respectively, and the light chain variable region comprises LCDR1, LCDR2, and LCDR3 of the amino acid sequences as set forth in SEQ ID NOs: 8-10, respectively.

2. The BTN3A-binding protein of claim 1, comprising:
a heavy chain variable region of the amino acid sequence as set forth in any one of SEQ ID NOs: 1, 3, 31-35, and 39-44, or an amino acid sequence with at least 90% identity thereto, and
a light chain variable region of the amino acid sequence as set forth in any one of SEQ ID NOs: 2, 4, 36-38, and 45-47, or an amino acid sequence with at least 90% identity thereto;
preferably,
1) comprising a heavy chain variable region of the amino acid sequence as set forth in any one of SEQ ID NOs: 1 and 31-35 or an amino acid sequence with at least 90% identity thereto, and a light chain variable region of the amino acid sequence as set forth in any one of SEQ ID NOs: 2 and 36-38 or an amino acid sequence with at least 90% identity thereto; or
2) comprising a heavy chain variable region of the amino acid sequence as set forth in any one of SEQ ID NOs: 3 and 39-44 or an amino acid sequence with at least 90% identity thereto, and a light chain variable region of the amino acid sequence as set forth in any one of SEQ ID NOs: 4 and 45-47 or an amino acid sequence with at least 90% identity thereto; and
more preferably,
1) comprising a heavy chain variable region of the amino acid sequence as set forth in SEQ ID NO: 1 or an amino acid sequence with at least 90% identity thereto, and a light chain variable region of the amino acid sequence as set forth in SEQ ID NO: 2 or an amino acid sequence with at least 90% identity thereto;
comprising a heavy chain variable region of the amino acid sequence as set forth in any one of SEQ ID NOs: 31-35 or an amino acid sequence with at least 90% identity thereto, and a light chain variable region of the amino acid sequence as set forth in SEQ ID NO: 36 or an amino acid sequence with at least 90% identity thereto;
comprising a heavy chain variable region of the amino acid sequence as set forth in any one of SEQ ID NOs: 31-35 or an amino acid sequence with at least 90% identity thereto, and a light chain variable region of the amino acid sequence as set forth in SEQ ID NO: 37 or an amino acid sequence with at least 90% identity thereto; or
comprising a heavy chain variable region of the amino acid sequence as set forth in any one of SEQ ID NOs: 31-35 or an amino acid sequence with at least 90% identity thereto, and a light chain variable region of the amino acid sequence as set forth in SEQ ID NO: 38 or an amino acid sequence with at least 90% identity thereto; or
2) comprising a heavy chain variable region of the amino acid sequence as set forth in SEQ ID NO: 3 or an amino acid sequence with at least 90% identity thereto, and a light chain variable region of the amino acid sequence as set forth in SEQ ID NO: 4 or an amino acid sequence with at least 90% identity thereto;
comprising a heavy chain variable region of the amino acid sequence as set forth in any one of SEQ ID NOs: 39-44 or an amino acid sequence with at least 90% identity thereto, and a light chain variable region of the amino acid sequence as set forth in SEQ ID NO: 45 or an amino acid sequence with at least 90% identity thereto;
comprising a heavy chain variable region of the amino acid sequence as set forth in any one of SEQ ID NOs: 39-44 or an amino acid sequence with at least 90% identity thereto, and a light chain variable region of the amino acid sequence as set forth in SEQ ID NO: 46 or an amino acid sequence with at least 90% identity thereto; or
comprising a heavy chain variable region of the amino acid sequence as set forth in any one of SEQ ID NOs: 39-44 or an amino acid sequence with at least 90% identity thereto, and a light chain variable region of the amino acid sequence as set forth in SEQ ID NO: 47 or an amino acid sequence with at least 90% identity thereto.

3. A BTN3A-binding protein, comprising a domain that specifically binds to BTN3A and comprises a heavy chain variable region and a light chain variable region, wherein:
the heavy chain variable region comprises HCDR1, HCDR2, and HCDR3 of the amino acid sequences as set forth in SEQ ID NOs: 17-19, respectively, and
the light chain variable region comprises LCDR1, LCDR2, and LCDR3 of the amino acid sequences as set forth in SEQ ID NOs: 20-22, respectively.

4. The BTN3A-binding protein of any one of claims 1-3, which is an anti-BTN3A antibody or an antigen-binding fragment thereof, wherein the antibody is preferably a rabbit-derived antibody, a chimeric antibody, a humanized antibody, or a fully human antibody; and the antigen-binding fragment is preferably an scFv, Fab, or F(ab')2 fragment.

5. The BTN3A-binding protein of any one of claims 1-4, which is an anti-BTN3A antibody or an antigen-binding fragment thereof that is engineered by humanization, backmutation, affinity maturation, removal of a T cell epitope, reduction of antibody deamidation, and/or reduction of antibody isomerization;
wherein preferably, in the human germline template used in the humanization engineering process, the heavy chain framework regions are derived from IGHV3-66, and the light chain framework regions are derived from IGkV1-27.

6. The BTN3A-binding protein of any one of claims 1-5, further comprising an Fc region of an immunoglobulin;
wherein preferably, the Fc region is the Fc region of human IgG1, human IgG2, human IgG3, or human IgG4; and
more preferably, the Fc region is the Fc region of human IgG1 with L234F, L235E, and/or P331S mutations, in which the mutated amino acid positions are defined according to the EU numbering system, and optionally, K446 is deleted.

7. The BTN3A-binding protein of any one of claims 1-3 and 5-6, comprising:
a heavy chain of the amino acid sequence as set forth in any one of SEQ ID NOs: 25, 27, 48-52, and 56-61, or an amino acid sequence with at least 80% or at least 90% identity thereto, and
a light chain of the amino acid sequence as set forth in any one of SEQ ID NOs: 26, 28, 53-55, and 62-64, or an amino acid sequence with at least 80% or at least 90% identity thereto;
preferably,
1) comprising a heavy chain of the amino acid sequence as set forth in any one of SEQ ID NOs: 25 and 48-52 or an amino acid sequence with at least 80% or at least 90% identity thereto, and a light chain of the amino acid sequence as set forth in any one of SEQ ID NOs: 26 and 53-55 or an amino acid sequence with at least 80% or at least 90% identity thereto; or
2) comprising a heavy chain of the amino acid sequence as set forth in any one of SEQ ID NOs: 27 and 56-61 or an amino acid sequence with at least 80% or at least 90% identity thereto, and a light chain of the amino acid sequence as set forth in any one of SEQ ID NOs: 28 and 62-64 or an amino acid sequence with at least 80% or at least 90% identity thereto; and
more preferably,
1) comprising a heavy chain of the amino acid sequence as set forth in SEQ ID NO: 25 or an amino acid sequence with at least 80% or at least 90% identity thereto, and a light chain of the amino acid sequence as set forth in SEQ ID NO: 26 or an amino acid sequence with at least 80% or at least 90% identity thereto;
comprising a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 53 or an amino acid sequence with at least 80% or at least 90% identity thereto;
comprising a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 54 or an amino acid sequence with at least 80% or at least 90% identity thereto; or
comprising a heavy chain of the amino acid sequence as set forth in any one of SEQ ID NOs: 48-52 or an amino acid sequence with at least 80% or at least 90% identity thereto, and a light chain of the amino acid sequence as set forth in SEQ ID NO: 55 or an amino acid sequence with at least 80% or at least 90% identity thereto; or
2) comprising a heavy chain of the amino acid sequence as set forth in SEQ ID NO: 27 or an amino acid sequence with at least 80% or at least 90% identity thereto, and a light chain of the amino acid sequence as set forth in SEQ ID NO: 28 or an amino acid sequence with at least 80% or at least 90% identity thereto;
comprising a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 62 or an amino acid sequence with at least 80% or at least 90% identity thereto;
comprising a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 63 or an amino acid sequence with at least 80% or at least 90% identity thereto; or
comprising a heavy chain of the amino acid sequence as set forth in any one of SEQ ID NOs: 56-61 or an amino acid sequence with at least 80% or at least 90% identity thereto, and a light chain of the amino acid sequence as set forth in SEQ ID NO: 64 or an amino acid sequence with at least 80% or at least 90% identity thereto.

8. The BTN3A-binding protein of any one of claims 1-7, which specifically binds to one or more of BTN3A1, BTN3A2, and BTN3A3, preferably BTN3A1, BTN3A2, and BTN3A3.

9. A polynucleotide encoding the BTN3A-binding protein of any one of claims 1-8;
wherein preferably, the polynucleotide is DNA or RNA.

10. A vector, comprising the polynucleotide of claim 9.

11. A host cell, comprising or expressing the polynucleotide of claim 9 or the vector of claim 10.

12. A method for preparing a BTN3A-binding protein, comprising:
expressing the polynucleotide of claim 9 or the vector of claim 10 in the host cell of claim 11, and isolating the expressed BTN3A-binding protein from the host cell; and
optionally further comprising a step of purifying the BTN3A-binding protein.

13. A pharmaceutical composition, comprising:
the BTN3A-binding protein of any one of claims 1-8, and
at least one pharmaceutically acceptable excipient, diluent, or carrier.

14. A method for treating or preventing cancer, comprising:
administering to a subject in need thereof a therapeutically or prophylactically effective amount of the BTN3A-binding protein of any one of claims 1-8, the polynucleotide of claim 9, the vector of claim 10, or the pharmaceutical composition of claim 13.

15. A method for activating γδ T cells and/or promoting cytokine release by γδ T cells, comprising:
administering to the γδ T cells or contacting the γδ T cells with the BTN3A-binding protein of any one of claims 1-8, the polynucleotide of claim 9, the vector of claim 10, or the pharmaceutical composition of claim 13, wherein the γδ T cells may be *in vivo* or *ex vivo;* and
the γδ T cells are preferably Vγ9Vδ2 T cells.
